# EUROPEAN PATENT APPLICATION

(11) **EP 2 206 774 A1**
(43) Date of publication of application: **14.07.2010**
(21) Application number: 08291236.1
(22) Date of filing: 23.12.2008
(51) Int. Cl.: C12N 5/077, C12N 5/0775

(54) **Muscle-derived cells having differentiation capacities**

(71) Applicant: Genzyme Corporation, Cambridge, MA 02142 (US); INSTITUT NATIONAL DE LA SANTE ET DE LA RECHERCHE MEDICALE, 75654 Paris Cedex 13 (FR)
(72) Inventor: Vilquin, Jean-Thomas, 77500 Chelles (FR); Vauchez, Karine, 75005 Paris (FR); Marolleau, Jean-Pierre, 80000 Amiens (FR)
(74) Representative: Paris, Fabienne

(57) **Abstract**

The present application relates to cell populations having differentiation capacities, which are obtainable by isolation from a muscle tissue, more particularly from a skeletal and/or cardiac muscle tissue, preferably from endomysial and/or cardiac tissue, more preferably from endomysial tissue. The cell populations of the invention comprise ALDH-positive cells, and notably have myogenic and/or adipogenic and/or osteogenic differentiation capacities.

The present application also relates to those of said cell populations, which comprise CD34-negative cells, and to those of said cell populations, which comprise CD34-positive cells. The cell populations of the invention, which comprise ALDH-positive cells and CD34-negative cells, notably have myogenic and/or osteogenic differentiation capacities. The cells populations of the invention, which comprise ALDH-positive cells and CD34-positive cells, notably have adipogenic and/or osteogenic differentiation capacities.

The present application also relates to the biotechnological and medical applications of these cell populations, notably in the field of cell and tissue therapy.

## Description

### FIELD OF THE INVENTION

The present application relates to cell populations, which have differentiation capacities, and which, prior to isolation, are naturally-occurring in muscle tissue(s). At the time of their isolation from muscle tissue(s), and before any step of cell proliferation and/or differentiation, the cell populations of the invention comprise ALDH-positive cells. The cell populations of the invention notably have myogenic and/or adipogenic and/or osteogenic differentiation capacities.
More particularly, the present application relates to those of said cell populations, which comprise CD34-negative cells, and to those of said cell populations, which comprise CD34-positive cells.
The cell populations of the invention, which comprise ALDH-positive and CD34-negative cells, notably have myogenic and/or osteogenic differentiation capacities.
The cells populations of the invention, which comprise ALDH-positive and CD34-positive cells, notably have adipogenic and/or osteogenic differentiation capacities.
The present application also relates to a cell obtainable by isolation from a cell population of the invention, and to cells and tissues, which are derivable from said cell populations of the invention, e.g., by culture and/or differentiation of said cells.
The present application also relates to the biotechnological and medical applications of the cell populations, cell and derivable cells and tissues of the invention, notably in the field of cell therapy and tissue therapy.

### BACKGROUND OF THE INVENTION

Aldehyde dehydrogenases (ALDHs) constitute a large and ancient family of intracellular enzymes involved in oxidation of aliphatic and aromatic aldehydes into the corresponding acids, thereby considered as general detoxifying enzymes eliminating toxic biogenic and xenobiotic aldehydes in humans. ALDH1 and ALDH3 isoenzymes are cytosolic, ubiquitously distributed, and especially allow resistance to anti-cancer drugs of the oxazaphosphorine family by their detoxification.

ALDH1 activity is known to be expressed by human hematopoietic progenitors. The first description was performed using antibodies (Kastan *et al*., 1990). It was confirmed and extended using fluorescent synthetic aminoacetaldehyde (AAA) substrates (Jones *et al.,* 1995; Storms *et al.* 1999), such as the dipyrrometheneboron difluoxide (BODIPY™) aminoacetaldehyde (AAA) substrate used in the Aldefluor® kit that is commercially-available from Stem Cell Technologies. Intracellular ALDH1 converts BOPIDY™-AAA (BAAA) into BOPIDY™-aminoacetate (BOPIDY™-AA; or BAA), which is retained intracellularly because of its net negative charge, which disallows free diffusion. The assay buffer (supplied with the Aldefluor® kit) contains a transport inhibitor, which prevents efflux of the BAA from the cells, which usually express this transporter. Because of the net negative charge of the BAA, and blockade of the transporter if any, cells expressing high levels of ALDH retain BAA and thus fluoresce. They can then be detected using the green fluorescence (FL1) channel of a standard flow cytometer. Flow cytometry and cell sorting allowed delineating specific cell populations with low side scatter and high (bright) ALDH1 activity (SSC^{lo}/ALDH^{br}) within human bone marrow, human umbilical cord blood, and human peripheral blood. These ALDH^{br}/SSC^{lo} cells are rare cells, with frequencies not exceeding 5% of the mononucleated cellular fraction of said hematopoietic fluids in non-pathologic conditions. Lineage-negative cell depletions and co-labeling with classical hematopoietic markers such as CD34, CD38, CD45 or CD133 refined the characterizations of sub-populations and their differentiation capacities *in vitro* and *in vivo.*
ALDH1 and ALDH3 are also known to be expressed by human tumor cell lines that are resistant to alkylating agents.

Alternate differentiation capacities have been attributed to bone marrow ALDH^{br}, cells towards mesenchymal and endothelial lineages depending on the culture conditions.

Information on ALDH1 expression in non-hematopoietic cells is scarce.

Immunofluorescence microscope analysis of fetal rat brain cells indicated that rat neural stem cells express ALDH1, whereas ALDH^{br}, cells are rare among rat neural progenitor cells. These results suggested that ALDH1 activity could be used to distinguish between neural stem and progenitor cells in rodent fetal neural tissue (Cai *et al.* 2004; Corti *et al.* 2006a; Corti *et al.* 2006b).

One group reported ALDH1 expression in CD45⁻ CD31⁺ human umbilical cord blood cells, which might represent endothelial cells (Hess *et al.* 2003).

Another group reported that a sub-set of endothelial progenitor cells from human umbilical cord blood expresses ALDH1 activity. These progenitor cells were not directly obtained by isolation from human umbilical cord blood, but were produced by *in vitro* culture of isolated cells. The results obtained with these *in vitro*-produced cells demonstrated that ALDH1-low endothelial progenitor cells are far more suitable than those with high ALDH1 activity to restore vascularization in ischemic tissue, because ALDH1-low endothelial progenitor cells possess a greater ability to proliferate and migrate compared to those with high ALDH1 activity and because ALDH1-low endothelial progenitor cells have, contrary to those with high ALDH1 activity, the capacity to up-regulate hypoxia-inducible factor proteins (Nagano *et al.* 2007).

ALDH1 activity has also been detected in a sub-population of cancer and normal human mammary epithelial cells. These results suggested that human normal mammary epithelial cells with ALDH1 activity might have lineage differentiation potential (mammary formation), and that human carcinoma mammary epithelial cells with high ALDH1 activity would have a self-renewal capacity, which would associate with poor prognosis (Ginestier *et al.* 2007).
However, further research into ALDH expression and activity in mammary epithelial cells is needed, as other ALDH isoenzymes might be involved in the dehydrogenase activity observed in these cells.

The so-called "stromal-vascular fraction" prepared from adipose tissue has been described to contain a sub-population of ALDH1-expressing cells (reaching up to 14% at initial isolation). When placed in culture, the whole cell preparation presents first an increase in proportion of cells expressing ALDH (reaching 90%) on first passages, then presents a decrease as the number of passages increases (down to 10% on passage 9) (Mitchell *et al.* 2006).

High ALDH1 activity is now considered a hallmark of cells harboring high myeloid, lymphoid, erythroid differentiation ability, and short and/or long-term reconstitution capacities *in vivo.*

Several cell types from the human skeletal muscle have been identified as having a stem/progenic potential, but some of them have been described as having a progenic potential suitable for myogenesis.

The satellite cells, which are located beneath the muscle fiber basal lamina, are currently considered as the main myogenic progenitors, allowing growth, homeostasis and regeneration. These quiescent cells act through activation and expansion into committed myoblasts. Their self-renewal is warranted by asymetric division. Human satellite cells are notably described as expressing the surface marker CD56 (NCAM).

Other adult stem/progenitor cells have been described in the skeletal muscle.

Side population (SP) cells, as defined by their capacity to exclude Hoechst dyes, constitute a rare population comprising undifferentiated cells, which have initially been identified in mice. Most studies on SP cells have been performed on murine (mouse) SP cells, although SP cells have been described in the muscle of a human fetus and a human adult in one case. The differentiation potential of SP cells is relatively limited as they are not capable of myogenesis by themselves: they require co-culture with myoblasts to undergo myogenic specification (Asakura *et al.* 2002).
Pericytes, maybe related to mesangioblasts as progenitors or progeny, wrap intramuscular vessels, express α-SMA, CD44, CD146, NG2, ALP, and present a myogenic differentiation capacity.
Myo-endothelial cells expressing CD146 and CD31 or CD56, CD34 and CD144 present myogenic differentiation capacities.

Finally, muscle CD133⁺ cells, which are located inside the muscle vessels, as well as circulating CD133⁺ cells may take part to muscle regeneration. SP cells, pericytes, and CD133⁺ cells progenitors are not restricted to the skeletal muscle compartment and may be considered as variants of ubiquitous tissue-specific stem cells.

Moreover, the differentiation of human cells towards specific structures *in vitro* (chondrocyte-, osteocyte-, adipocyte-like, neural-like cells, smooth muscle cells) has been observed in dedicated media but could not always be attributed to an identified cellular progenitor.

### SUMMARY OF THE INVENTION

It is believed that the presence of cell populations having differentiation capacities and comprising ALDH-positive cells has never been described in a muscle, more particularly in a striated muscle, still more particularly in a skeletal and/or cardiac muscle, most particularly in the endomysium of a skeletal muscle and/or in a cardiac muscle, preferably in the endomysium of a skeletal muscle.

Based on a measurement of ALDH activity, which does not require any step of *in vitro* culture, the inventors have isolated a well-defined population of ALDH-positive cells from bulk dissociated muscle biopsies. These cells have the features of progenitors, more particularly of primitive progenitors. This ALDH⁺-containing cell population notably has myogenic, adipogenic and osteogenic differentiation capacities. More particularly, contrary to the Side Population (SP) cells, this ALDH⁺-containing cell population has the capacity of undergoing myogenesis in the absence of co-cultured myoblasts or co-cultured cardiomyocytes.

The inventors isolated two well-defined sub-populations within the ALDH⁺-containing cell population.
One of these two sub-populations comprises cells, which are ALDH-positive and CD34-negative, and has strong myogenic differentiation capacities *in vitro* and *in vivo*, as well as strong osteogenic differentiation capacities.

The other sub-population comprises cells, which are ALDH-positive and CD34-positive, and has strong adipogenic and osteogenic differentiation capacities.

Whereas aldehyde dehydrogenase (ALDH) activity is currently considered to be one hallmark of some populations of human primitive hematopoietic cells from bone marrow, peripheral and umbilical cord blood, the inventors bring the demonstration that ALDH-positive cells are present in muscles including human muscles, more particularly in striated muscles, still more particularly in skeletal and/or cardiac muscles, even still more particularly in the endomysium of a skeletal muscle and/or in a cardiac muscle, most particularly in the endomysium of a skeletal muscle, and that these cells have strong differentiation capacities, including myogenic, adipogenic and osteogenic differentiation capacities.

CD34 membrane labeling further discriminated two sub-populations: a sub-population, which comprises ALDH⁺/CD34⁻ cells, and a sub-population, which comprises ALDH⁺/CD34⁺ cells.
Both sub-populations generally contained some CD90⁺ cells, but were devoid or quasi-devoid of CD31⁺, CD45⁺ and CD56⁺ cells, which were identified in the ALDH-negative populations.
The ALDH⁺/CD34⁺ containing sub-population generally comprised a few CD44⁺ and CD140b⁺ cells, and developed *in vitro* as a heterogeneous population of CD56⁻ cells able to differentiate in adipoblasts.
The ALDH⁺/CD34⁻ containing sub-population generally comprised some CD44⁺ and CD105⁺ cells, and developed *in vitro* as a highly enriched population of CD56⁺ myoblasts able to form myotubes.
Moreover, the ALDH⁺/CD34⁺ containing sub-population and the ALDH⁺/CD34⁻ containing sub-population presented distinct functional abilities *in vivo.* Upon intramuscular transplantation in SCID mice, ALDH⁺/CD34⁻ cells survived, proliferated and participated to muscle regeneration, whereas ALDH⁺/CD34⁺ cells did not.
Both ALDH⁺/CD34⁻ and ALDH⁺/CD34⁺ containing sub-populations have osteogenic differentiation capacities.

Hence, the inventors demonstrate that ALDH is a marker of a muscle cell sub-population, which is distinct from any of the previously-known stem or progenitor cells, and which harbors strong differentiation capacities, including myogenic and/or adipogenic and/or osteogenic differentiation capacities.

The cells of the invention are very useful for various biotechnological and medical applications, more particularly for cell or tissue therapy.

Therefore, the invention relates to these cells, as well as to a process for the production of such cells, and to the use of an ALDH substrate for the detection of cells in a sample comprising muscle cells, including the subject-matter as defined in the claims.
The present invention further relates to cell populations derivable by cell differentiation, to a process for the production of myocytes, myotubes, muscles, adipocytes, adipose tissues, osteocytes, bones, chondrocytes, cartilage, and to the biotechnological and medical applications of the cells of the invention, including cell or tissue therapy compositions and the treatment of diseases or conditions.

### BRIEF DESCRIPTION OF THE FIGURES

Some of the figures, to which the present application refers, are in color. The application as filed contains the color print-out of the figures, which can therefore be accessed by inspection of the file of the application at the patent office.

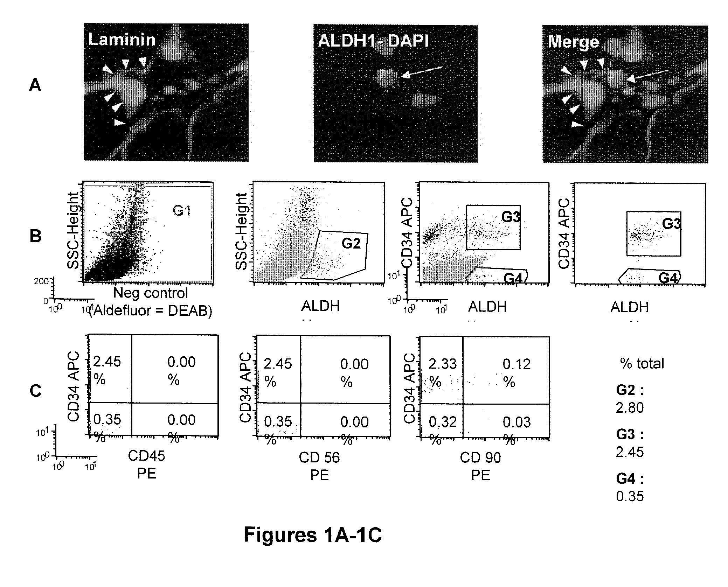

### Figures 1A, 1B, 1C: Immunohistological and flow cytometric characterization of ALDH-positive cells in human skeletal muscle.

Figure 1A: Immunohistofluorescence localization of ALDH1-positive cell in human skeletal muscle. The anti-laminin antibody delineated the muscle fiber basal lamina (red, arrow heads) and the anti-human ALDH-1 antibody stained an ALDH1-positive cell (green). Nuclei are stained in blue with DAPI. A merge image indicated the endomysial localization of ALDH1-positive cell (arrow). Original magnification X100.
Figure 1B: Representative flow cytometric analysis of ALDH activity expressed by cells from enzymatically-dissociated human muscle (FacsCalibur apparatus, BD). Cells released from human biopsies were incubated with Aldefluor® substrate and the specific inhibitor of ALDH (DEAB) to establish the baseline fluorescence of the cell, and Gate 1 (G1) was defined to exclude debris (first panel). Based on G1, ALDH^{br} cells incubated in absence of DEAB presented a shift of fluorescence, thus defining the population in Gate 2 (G2) (second panel).
   ALDH^{br}/SSC^{lo} (abbreviated "SMALD") cell sub-populations were then discriminated by co-expression of CD34. Based on G2, SMALD/34⁺ (G3) and SMALD/34⁻ (G4) were clearly discriminated by the level of APC-conjugated CD34 labeling (third and fourth panels), and the percentages of cells in each gate is given relatively to the total number of cells included in G1.
Figure 1C: Based on G2 parameters defined for total ALDH activity, double labeling for APC-conjugated CD34 and PE-conjugated CD45 (left panel), CD56 (middle panel) or CD90 (right panel), enabled a dot-plot phenotypic characterization of both SMALD sub-populations. Percentages in plots relate to the total number of cells included in G1.

### Figures 2A, 2B: Cell sorting and phenotypic analysis in vitro.

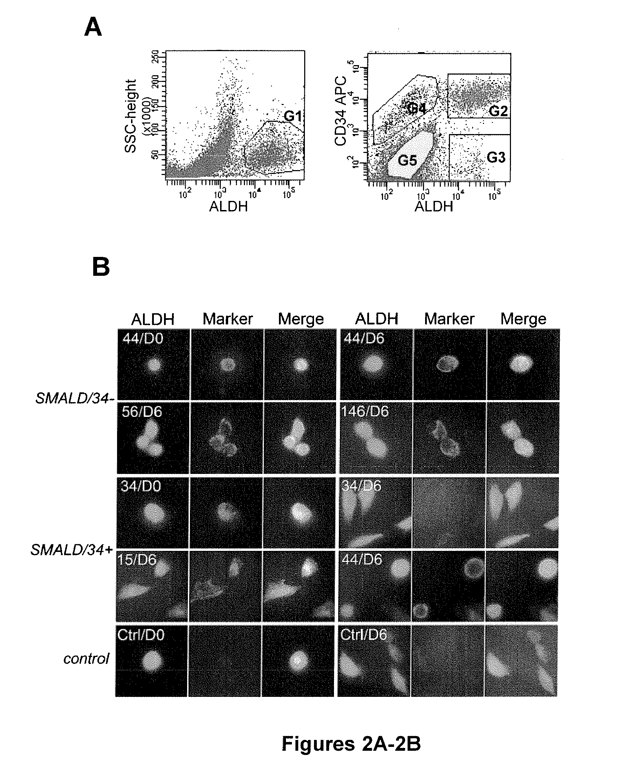

Figure 2A: Flow cytometric analysis, gating and sorting of dissociated muscle sub-populations (Vantage Se DIVA apparatus). Cells were labeled for ALDH activity and CD34 expression. The shift of fluorescence defined the population in G1 (left panel) presenting high ALDH activity. The following gates defined the sub-populations on the basis of ALDH activity and APC-conjugated CD34 labeling: G2: SMALD/34⁺, G3: SMALD/34⁻ , G4: single positive CD34⁺ (spCD34), and G5: Double Negative (DN) cells (right panel). G2 and G3 were first determined on G1.
Figure 2B: Immunocytofluorecence analysis of SMALD/34⁻ and SMALD/34⁺ cells. SMALD cells were labeled for expression of ALDH by incubation with Aldefluor® (green) and extracellular markers (PE-conjugated, red) 18h (D0) and 6 days (D6) after plating. Nuclei were stained by DAPI (blue). SMALD/34⁻: some cells only expressed CD44 on D0, while numerous cells expressed CD44, CD56 and CD146 on D6.
   SMALD/34⁺: the cells only expressed CD34 on D0, while on D6 numerous cells expressed CD15 or CD44, but not CD34. Controls were obtained on D0 and D6 by omission of the primary antibodies. Original magnification X60.

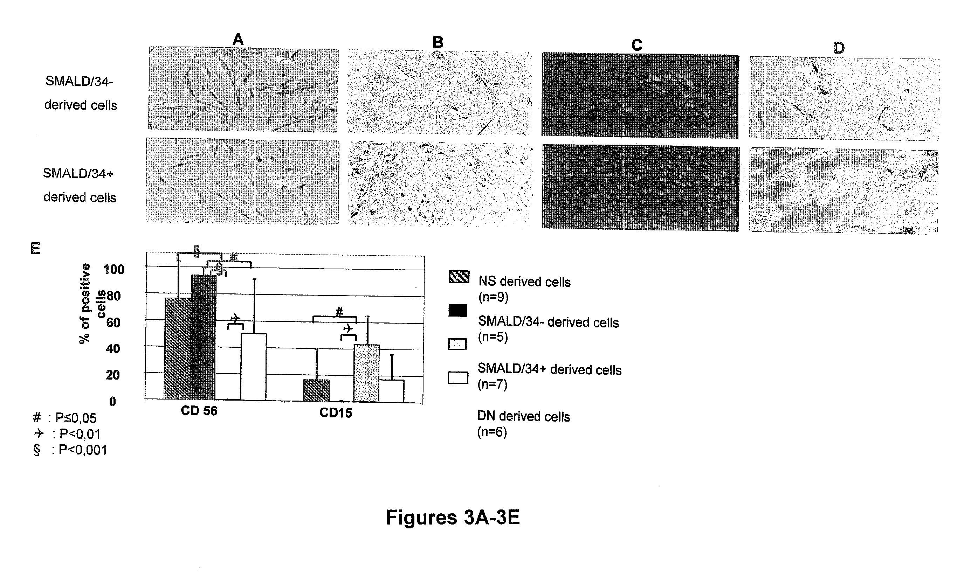

### Figures 3A, 3B, 3C, 3D, 3E: In vitro analysis of SMALD/34- and SMALD/34⁺-derived cells.

Figures 3A-3D: Phase contrast pictures illustrated SMALD/34⁻ and SMALD/34⁺ cells expanded in proliferation for 2 or 3 passages (Figure 3A) and shifted to differentiation media (Figures 3B-3D). Upon sorting and expansion of SMALD fractions, the cells were grown in specific media allowing myogenic or adipogenic differentiations *in vitro.* SMALD/34⁻ derived cells underwent myogenesis (Figure 3B) as assessed by formation of myotubes (phase contrast) expressing fast MHC isoform (Figure 3C, red). Nuclei were stained with DAPI (Figure 3C, blue). SMALD/34⁺ derived cells underwent adipogenesis (Figure 3D) as assessed by the accumulation of lipid droplets of various sizes. Original magnifications: X10 except for SMALD/34⁺ in Figure 3D, X30.
Figure 3E: Phenotypic characterizations were performed by flow cytometry for CD56 and CD15 membrane markers on expanded Non Sorted ("NS"), SMALD/34', SMALD/34⁺ and Double Negative ("DN") populations [left side of the graph: CD56 marker; right side of the graph: CD15 marker; for each marker, bars are shown in the following order, from left to right: NS-derived cells, SMALD/34⁻-derived cells, SMALD/34⁺-derived cells, DN-derived cells]. Most SMALD/34⁻ derived cells expressed CD56 in culture, but not CD15, while half SMALD/34⁺-derived cells expressed CD15 but never CD56. CD56⁺ cells also originated from Non Sorted ("NS") and Double Negative ("DN") fractions. n = numbers of biopsies analyzed.

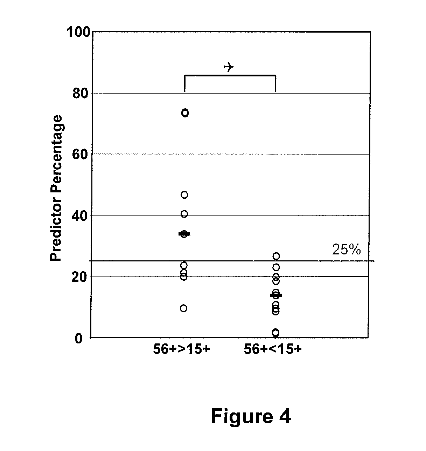

### Figure 4: Predictive value of the Predictor percentage (PP).

Flow cytometric analysis of Aldefluor® and CD34-APC staining on dissociated muscle cells (n=20 biopsies) enabled initial calculation of PP value for each biopsy. The PP values corresponded to the percentages of SMALD/34⁻ cell number in the total SMALD cell number. Following expansion in culture (2-3 passages), cells were characterized for CD56 and CD15 expression by FACS, and were divided in two groups according to respective proportions of cells expressing CD56 or CD15: [CD56+ > CD15+] and [CD56+ < CD15+]. In each group, biopsies (circles) were ranked as functions of their PP, with median PP indicated by black dash. The threshold PP value calculated at 25% (red line) statistically delineated the predictive yield of a biopsy.

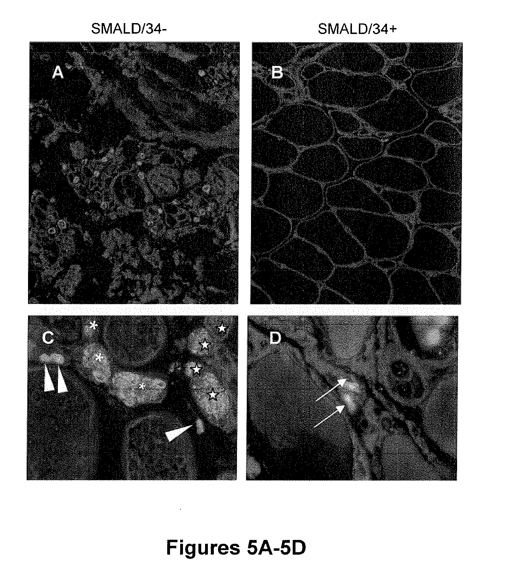

### Figures 5A, 5B, 5C, 5D: In vivo transplantation of SMALD/34⁻ and SMALD/34⁺ sorted cells.

Sorted cells were injected in *tibialis anterior* of SCID mice, pre-treated by local irradiation and notexin injection. Immunofluorescence microscopy was performed on transversal sections of muscles removed 4 weeks after cell transplantation. Human nuclei were labeled by anti-human lamin A/C protein monoclonal antibody (red). Muscle fiber basal lamina was delineated by anti-laminin staining (blue). Human mitochondria were labeled by anti-human COX2 protein (green; Figures 5C, 5D) and allowed detection of fibers regenerated by donor SMALD cells. Numerous SMALD/34-cells were observed at injection sites (Figure 5A) and participated to muscle formation as illustrated by the presence of human mitochondria within muscle fibers containing (white *) or not (white *) human nuclei (Figure 5C). Only a few SMALD/34⁺ cells were observed at injection sites (Figure 5B) and remained in the endomysium (Figure 5D, arrow). Original magnifications: X25 (Figures 5A, 5B), X100 (Figure 5C, 5D).

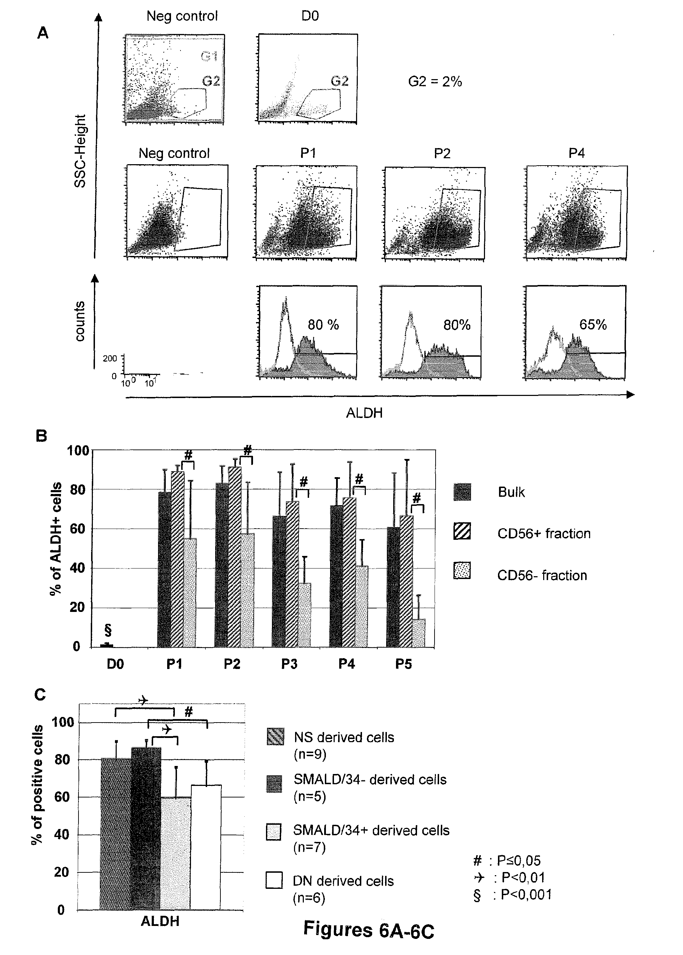

### Figures 6A, 6B, 6C: Assessment of ALDH activity in cultured cells.

Flow cytometric evaluation of ALDH activity was performed using Aldefluor® on human muscle dissociated cells, without sorting, as a function of passage number (Figure 6A). Although a few cells initially expressed ALDH, their number raised dramatically in culture and was maintained for at least 4 passages. Coupled to ALDH staining, flow cytometric analysis of CD56 expression by non sorted cells in culture allowed comparing the levels of ALDH expression in CD56⁺, CD56⁻ and un-fractionated cells. The CD56⁻ fraction contained the lowest number of ALDH^{br} cells (Figure 6B). ALDH activity was assessed in cultures derived from the populations of sorted cells (Figure 6C). The cultures grown from SMALD/34⁺ and DN fractions contained significant lower numbers of ALDH^{br} cells.

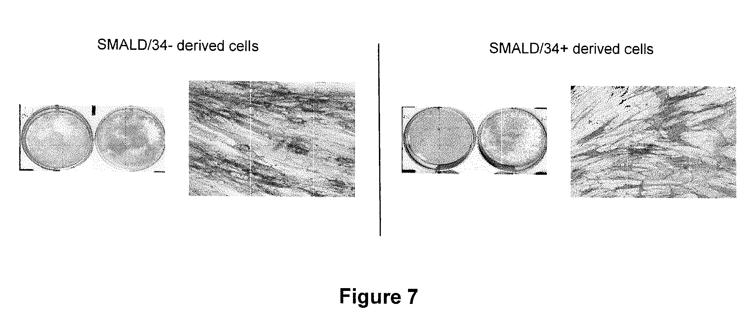

### Figure 7: In vitro osteogenic differentiation abilities of cells derived from sorted SMALD/34⁻ and SMALD/34⁺ populations.

Upon sorting and expansion of SMALD/34⁻ and SMALD/34⁺ populations (left and right panels, respectively), the cells were grown in osteogenic differentiation medium. Both populations underwent osteogenesis as assessed by ALP expression (purple) and mineralization (red). Optical view (numeric picture) and microscopic view (original magnification X10).

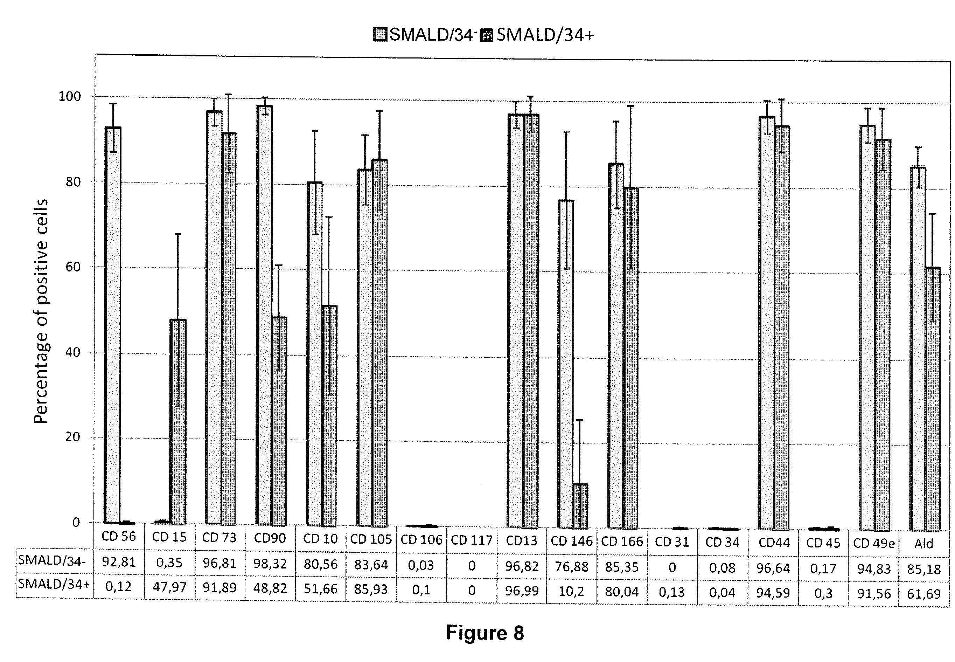

### Figure 8: Phenotypic (flow cytometry) analyses of SMALD/34⁻-derived cell cultures (left-hand bars in Figure 8) and of SMALD/34⁺-derived cell cultures (right-hand bars in Figure 8)

Analysis of CD10, CD13, CD15, CD31, CD34, CD44, CD45, CD49e, CD56, CD73, CD90, CD105, CD106, CD117, CD146, CD166 expression by SMALD/34⁻ and SMALD/34⁺-sorted cells after culture on proliferation medium.

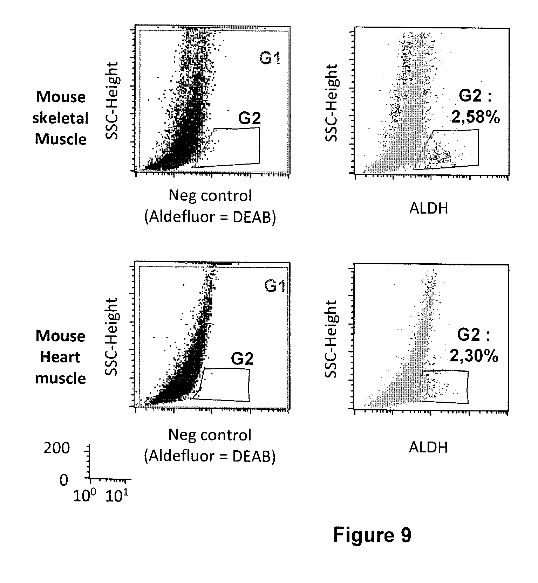

### Figure 9: ALDH activity in murine skeletal and cardiac muscle cells

Flow cytometric evaluation of ALDH activity was performed using Aldefluor® on murine skeletal and murine cardiac muscle cells.

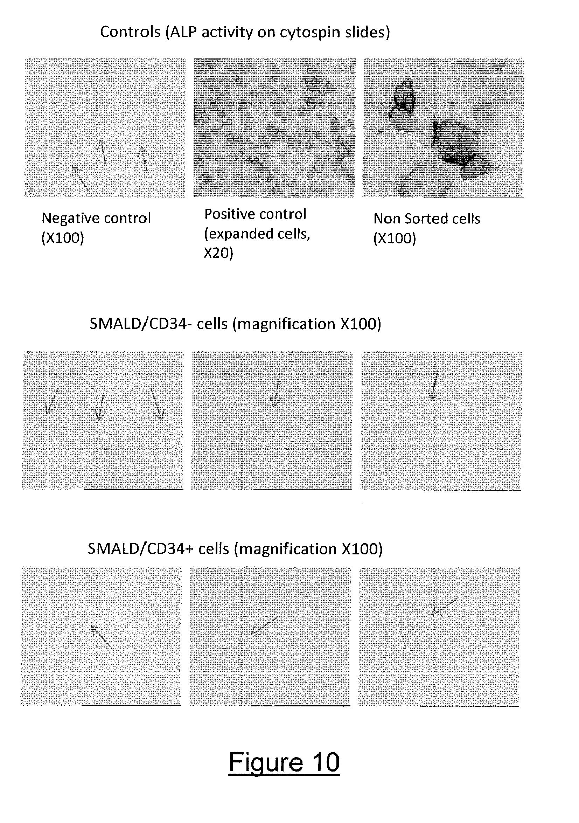

### Figure 10: SMALD/34⁻ and SMALD/34⁺ populations are ALP-negative.

Control cells, sorted cells, and non-sorted cells, were centrifuged on glass slides and assayed for ALP activity by incubation with NBT/BCIP. No labeling was observed in absence of the reagent (top panel, left), while a light to dark staining was obtained from control cells and whole non-sorted cells (top panel, middle and right). On the opposite, no ALP staining could be observed upon assay of SMALD/34- or SMALD/34+ sorted cells (middle and low panel). Magnifications are indicated on the picture.

### DETAILED DESCRIPTION OF THE INVENTION

The present application relates to the subject-matter as defined in the claims as filed and as herein described.

The present application notably relates to cell populations having differentiation potentials, to a process enabling to produce or isolate them, to cell populations obtainable by this process, to a cell obtainable by isolation from a cell population of the invention, to the cell populations or cells obtainable by differentiation of said cell population or cell, as well as to the biotechnological and medical applications of the cell populations or cell of the invention.

The present application notably relates to a cell population obtainable by isolation from muscle tissue(s).

### Cell populations comprising ALDH-positive cells:

A cell population of the invention comprises at least 50%, preferably at least 60%, more preferably at least 70%, still more preferably at least 80%, still even more preferably at least 90%, most preferably at least 95%, still most preferably at least 98%, even still most preferably at least 99% of ALDH-positive cells, for example 100% of ALDH-positive cells.
For the sake of conciseness, this population is herein referred to as ALDH⁺-containing cell population.
Therefore, an ALDH⁺-containing cell population of the invention mainly comprises ALDH-positive cells (at least 50% of ALDH-positive cells, as above-mentioned), but may nevertheless also contain ALDH-negative cells, although at less than 50% of the total number of cells contained in the cell population.

Preferably, said ALDH is ALDH1 and/or ALDH3, more preferably ALDH1, most preferably ALDH1A1.

The present application also relates to a process for the production or for the isolation of said ALDH⁺-containing cell population.

The process of the invention produces a cell population that is enriched in ALDH-positive, preferably ALDH1-positive and/or ALDH3-positive, more preferably ALDH1-positive cells, most preferably ALDH1A1-positive cells. The process of the invention comprises enriching a cell population, said cell population being obtainable or obtained from muscle tissue(s), in ALDH-positive, preferably ALDH1-positive and/or ALDH3-positive, more preferably ALDH1-positive, most preferably ALDH1A1-positive cells.

More preferably, said ALDH1-positive cells comprise ALDH1A-positive cells, most preferably ALDH1A1-positive cells.

In the present application, all the terms are given their respective ordinary meaning within the relevant field.

The term "progenitor" is used in its widest sense, i.e., as including stem cells, as well as multipotent and unipotent cells. Nevertheless, the term "progenitor" preferably means multipotent or unipotent cells, more preferably multipotent cells.

By enriching a cell population in a certain type of cells, it is herein meant any means, which enables to increase the number of said certain type of cells that are initially contained in said cell population.
An enrichment produces an enriched cell population, which comprises said certain type of cells at a cell percentage within the total cell population that is higher than its initial cell percentage prior to enrichment.
An enriched cell population comprises a proportion of said certain type of cells that is higher than the proportion that was initially contained prior to enrichment.

Enriching may be performed either by positive sorting or by negative sorting or by selecting by means of cell culture conditions.
Positive sorting notably encompasses the collection of those cells that are of the desired cell type, e.g., using flow cytometry and/or magnetic beads, as well as positive selection by means of favorable cell culture conditions. An illustrative enrichment process is described in WO 01/94555 and its US counter-part patent application(s) and/or patent(s) (e.g., US 2004 0043008 A1; US 2006 0088508 A1).
Negative sorting notably encompasses depleting said initial cell population from those cells that are not of the desired cell type, e.g., by lyzing or otherwise destructing or "killing" said cells that are not of the desired cell type or by negatively selecting by means of unfavorable cell culture conditions. Therefore, the process of the invention may alternatively, or complementarily and/or additionally comprise depleting a cell population, which comprises muscle cells, in those of its cells that are ALDH-negative, preferably ALDH1-negative and/or ALDH3-negative, more preferably ALDH1-negative, most preferably ALDH1A1-negative.

Said enrichment can be performed up to a certain cell proportion, e.g., up to a proportion at least 50% of the cells of said certain type in the enriched cell population. In the present application, the term "enriching" encompasses any enrichment proportion, but preferably any enrichment proportion of at least 50%, preferably at least 60%, more preferably at least 70%, still more preferably at least 80%, still even more preferably at least 90%, most preferably at least 95%, still most preferably at least 98%, even still most preferably at least 99% up to or more.
Therefore, the process of the invention produces a population of cells having differentiation capacities, which are at any purity level, but preferably at any purity level of at least 50%, preferably at least 60%, more preferably at least 70%, still more preferably at least 80%, still even more preferably at least 90%, most preferably at least 95%, still most preferably at least 99% or more.

Alternatively, the process of the invention produces a population of cells, which is enriched by at least two fold, three fold, five fold, ten fold, twenty fold, forty fold, forty-five fold or more.
By ALDH, it is herein intended aldehyde dehydrogenase (EC 1.2.1.3).

By ALDH+, ALDH⁺ or ALDH-positive, it is herein meant an aldehyde dehydrogenase presence or activity, more particularly an aldehyde dehydrogenase presence or activity that is superior to the one that would be detected on a negative control. In an assay for the detection of ALDH activity, a negative control could be performed i.e., under the same conditions but in the presence of an inhibitor of the aldehyde dehydrogenase activity under detection (e.g., 4-(diethylamino)benzaldehyde (DEAB), when ALDH1 activity is detected and/or measured).

The ALDH family is known to comprise at least 19 putatively functional genes (Vasiliou and Nebert, 2005).
Each isoenzyme family is identified by an arabic number, e.g., ALDH1, ALDH2, ALDH3, ALDH4, ALDH5, ALDH6, ALDH7, ALDH8, ALDH9, ALDH16, ALDH18.
Each family comprises several sub-families (which are identified by a letter; e.g., ALDH1A), optionally followed by an Arabic number (which denotes the individual gene within the sub-family; e.g., ALDH1A1).

In the present application, ALDH preferably is ALDH1 and/or ALDH3, more preferably ALDH1, still more preferably ALDH1A, most preferably ALDH1A1.

Human ALDH1A1 protein sequence is available under accession number NP_000680 (NCBI; Entrez Protein); ENSP00000366138 or ENSP00000297785 (EBI, WTSI; Ensembl-, ProtView); or P00352 (EBI, SIB, PIR; UniProt).
*Mus musculus* ALDH1A1 protein sequence is available under accession number NP_038495 (NCBI); ENSMUSP00000084918 or ENSMUSP00000066920 (BII; WTSI); or P24549 (EBI; SIB; PIR).

Methods to detect an ALDH presence or activity, more particularly an ALDH1 and/or an ALDH3 presence or activity, are known in the art.

Anti-ALDH antibodies, including anti-ALDH1 and/or anti-ALDH3-antibodies, are available to the person of ordinary skill in the art either commercially or from Microorganism Deposit Institutions. The person of ordinary skill in the art can also produce anti-ALDH monoclonal antibodies.

Enrichment of ALDH-containing cells can be performed by any means that are found appropriate by the person of ordinary skill in the art, preferably by cell sorting, e.g., by flow cytometry, with or without prior magnetic bead sorting. At least in certain circumstances, ALDH-containing cells may be presumed to have an ALDH activity, i.e., to be ALDH-positive.

To detect an ALDH activity, it is however recommended to detect the presence of an enzyme that is capable of catalyzing the oxidation of aldehyde, e.g., the oxidation of an aldehyde to a carboxylic acid.
Aldehyde substrates and means to detect their oxidation, e.g., by detection of the fluorescence emitted by the oxidized substrate, are available to the person of ordinary skill in the art.
Means to detect an ALDH activity notably comprises the use of a fluorescent acetaldehyde, more particularly a fluorescent aminoacetaldehyde as a substrate, the oxidation of which leads to fluorescent acetate or fluorescent aminoacetate, respectively.
In accordance with the present invention, one of the preferred means to detect an ALDH activity comprises the use of dipyrrometheneboron difluoride (BODIPY™) aminoacetaldehyde (AAA) as a substrate. Intracellular ALDH activity, more particularly intracellular ALDH1 and/or ALDH3 activity, converts dipyrrometheneboron difluoride aminoacetaldehyde (BODIPY™-AAA; or BAAA) into dipyrrometheneboron difluoride aminoacetate (BODIPY™-AA; or BAA), which is retained intracellularly because of its net negative charge, which disallows free diffusion.
The fluorescence emitted by the cells can be detected and/or measured by any means that is found appropriate by the person of ordinary skill in the art, e.g., by flow cytometry.
A negative control can be made by using 4-(diethylamino)benzaldehyde (DEAB) as an ALDH1 inhibitor.

The Aldefluor® kit, which is commercially-available from StemCell Technologies, provides such means (StemCell Technologies; 400-570 - 7th Avenue W.; VANCOUVER; Bristish Columbia V5Z 1B3; CANADA). Said kit does not contain B-AAA, but a precursor form thereof, which has the advantage of being more stable than B-AAA at room temperature. Said precursor is BODIPY™-aminoacetaldehyde diethyl acetal (BODIPY™-AAA-DA; or BAAA-DA). Exposure to acid converts BAAA-DA into B-AAA.

Said kit further contains the DEAB inhibitor, as well as detailed instructions on how to perform the ALDH activity detection assay, to which the person of ordinary skill in the art may refer.
Said kit further contains an assay buffer, which contains a transport inhibitor. This transport inhibitor helps in limiting or preventing the efflux of BAA from the cells, which are equipped with such a promoter.
Said kit has been optimized for use with human peripheral blood and leukapheresis products. This kit is also known to be compatible with bone marrow and umbilical cord blood samples. The present inventors demonstrate that this kit is also compatible with muscle samples, preferably as described in the examples.
Therefore, the present invention also relates to the use of a fluorescent aldehyde ALDH substrate, more particularly of BAAA, for the detection of an ALDH activity or presence, more particularly of an ALDH1 and/or ALDH3 activity or presence, preferably an ALDH1 activity or presence, in cell(s) of muscle tissue(s) including human muscle tissue(s), more particularly of striated muscle tissue(s) including human striated muscle tissue(s), preferably of skeletal and/or cardiac muscle tissue(s) including human skeletal and/or cardiac muscle tissue(s), more preferably in cell(s) of the endomysium of a skeletal muscle and/or in cells of cardiac muscle tissue(s) including human skeletal muscle endomysium and/or human cardiac muscle tissue(s), most preferably in cells of skeletal muscle endomysium including human skeletal muscle endomysium.

In the present application, cells having an ALDH activity are more particularly defined as BAAA-stained cells, whose fluorescence is greater than that exhibited by at least 99%, preferably at least 99.9%, more preferably at least 99.95% of the cells exposed to DEAB.

In accordance with the present invention, the enrichment in ALDH-positive cells advantageously is up to a proportion of at least 50%, preferably of at least 60%, more preferably of at least 70%, still more preferably of at least 80%, still even more preferably of at least 90%, most preferably of at least 95%, still most preferably at least 99% of said ALDH-positive cells in the enriched cell population.

Said muscle tissue can be of any of the three types of muscle cells, i.e., said muscle tissue can be skeletal muscle tissue and/or cardiac muscle tissue and/or smooth muscle tissue.

Preferably, said muscle tissue is a striated muscle tissue, more particularly a skeletal muscle tissue and/or a cardiac muscle tissue.

Prior to the enrichment step of the process of the invention, the initial cell population may comprise cells from a cell line; or it may comprise cells, which have been collected from an animal.
Preferably, said initial cell population comprises cells, which have been collected from a living or dead animal, e.g., by biopsy, most preferably from a human.

Any type of muscle samples can be used in the process of the invention.
Reference is made to the reference manual entitled "Atlas of Human Anatomy", 4th Edition; Frank H. Netter, MD.; Anne Lenehan Ed., Elsevier Saunders, Philadelphia, Pennsylvania, USA; ISBN # 978-0-683-30492-3.
Advantageously, the process of the invention can be implemented on a standard muscle biopsy, such as a biopsy, which has been collected from the muscle of a human or a non-human animal.
Illustrative muscle biopsies comprise biopsy from a muscle of the head and/or neck (such as from the mouth, the tongue, the soft palate, the pharynx, the larynx, the cervical vertebra), from a muscle of the torso (such as from the back, the chest, the abdomen, the pelvis, the perineum, the heart), from a muscle of the upper limbs (such as from vertebral column, the thoracic walls, the shoulder, the arms, the forearms, the hand), from a muscle of the lower limb (such as from the iliac region, the buttocks, the thigh, the leg, the foot).
Preferred muscle biopsies are from the thigh (more preferably from the *fascia lata* tensor muscle), from the paravertebral muscle, or from the heart muscle.

When the initial cell population originates from collection from an animal, it is most usually collected under the form of a sample comprising a fragment of muscle tissue. To facilitate the enrichment step, said fragment of muscle tissue is advantageously dissociated, whereby a population of individual cells is obtained.
Techniques for dissociation of a muscle tissue are known in the art. For example, the dissociation of a muscle tissue may comprise mincing the muscle tissue and digestion by enzymatic treatment of the minced muscle tissue, e.g., using collagenase (e.g., for 1h at 37°C) and trypsin-EDTA (e.g., at 0.25%, for 20 min at 37°C). The cell suspension thereby obtained may then be filtered to collect the dissociated cells. Illustrative techniques are also described in WO 01/94555 and its US counter-part patent application(s) or patent(s) (e.g., US 2004 0043008 A1; US 2006 0088508 A1).

Said samples or biopsies may be fresh samples or biopsies.
If desired or required, said samples or biopsies may be kept under refrigeration or freezing (either before or after dissociation of the cells) until use. Therefore, the samples or biopsies to be implemented in the process of the invention can be frozen samples or biopsies (to be thawed before use), or frozen and thawed samples or biopsies.

Said initial cell population may comprise more than one muscle cell type, e.g., skeletal muscle cells and cardiac muscle cells, or skeletal muscle cells and smooth muscle cells, or cardiac cells and smooth muscle cells, or skeletal muscle cells and cardiac muscle cells and smooth muscle cells.

Advantageously, the process of the invention can be implemented on an initial cell population, which comprises a muscle cell type, i.e., skeletal muscle cells (preferably endomysial cells) or cardiac cells or smooth muscle cells as the muscle type. Indeed, the process of the invention can advantageously be implemented on a sample as small as a standard muscular biopsy, i.e., a sample of muscular tissue of about 0.1-10 g or more, e.g., 0.3-4 g, which has been collected from one or several human(s) or from one or several non-human animal(s).

A muscle tissue is made of various muscle cell sub-types. For example, a skeletal muscle comprises blood-vessel forming cells, blood vessel-associated cells, motoneuron axon-associated cells, myelinating cells, extracellular matrix-producing cells, basal lamina-producing cells, stromal cells, endomysial cells, endothelial cells, angiogenic cells, myocytes, myogenic cells, syncitial muscle cells. When the muscle has been collected from an animal, the collected sample further contains blood cells.

Therefore, said initial cell population may, and will more often comprise more than one muscle cell sub-type, e.g., it may comprise blood-vessel forming cells, blood vessel-associated cells, motoneuron axon-associated cells, myelinating cells, extracellular matrix-producing cells, basal lamina-producing cells, stromal cells, endomysial cells, endothelial cells, angiogenic cells, myocytes, myogenic cells, syncitial muscle cells.

Said initial cell population may comprise one or several other cell type(s) in addition to the muscle cell type(s), such as e.g., blood cells. Cell types that do not derive from muscle may also be encountered when the initial cell population originates or derives from a sample or biopsy, for example a sample or biopsy comprising vascular tissue(s) and/or aponevrotic tissue(s) and/or adipocyte tissue(s) in addition to muscle tissue(s).

In the present application, all the terms, more particularly terms such as "muscle", "muscle cells", "muscle tissue", "skeletal muscle", "endomysium", "cardiac muscle", "smooth muscle", are given their respective ordinary meaning within the relevant field, more particularly within the field of muscle physiology.

Without limitation, the invention relates to cell populations obtainable by isolation from one or more of three types of muscle tissues: skeletal muscle tissue and/or smooth muscle tissue and/or cardiac muscle tissue, preferably skeletal muscle tissue and/or cardiac muscle tissue, more preferably an endomysial tissue of a skeletal muscle and/or a cardiac muscle tissue, most preferably an endomysial tissue of a skeletal muscle.

Without limitation as to mechanisms, the following paragraphs are intended to provide context as to certain muscle biology.

A skeletal muscle is a striated muscle of voluntary type, which is anchored by tendons to bone. It is used to affect skeletal movement, such as locomotion, motricity as well as breathing, and in maintaining posture.

A smooth muscle is an "involuntary muscle", which is found within the walls of organs and structures such as the esophagus, stomach, intestines, bronchi, uterus, urethra, bladder, blood vessels, skin.

A cardiac muscle is also an "involuntary muscle", but is more akin in structure to skeletal muscle in the sense that it is a striated and not a smooth muscle. A cardiac muscle is found only in the heart.

In the present application, the term "skeletal muscle" does not encompass a heart muscle, although both types of muscles are striated muscles.

The schematic structure of a skeletal muscle is as follows.
Each muscular fiber of a skeletal muscle is a syncitial muscle cell containing from two to several thousands nuclei. Each muscular fiber of a skeletal muscle is sheathed by a membrane that is termed the basal lamina, which is in turn surrounded by a layer of connective tissue that is termed the endomysium.
The "muscular fiber-basal lamina-endomysium" units are organized in fascicules. Each fascicule is surrounded by a layer of connective tissue termed the perimysium.
Each skeletal muscle comprises several of such "fascicule-perimysium" units. A layer of connective tissue termed the epimysium sheaths the whole group of fascicule-perimyium units, thereby forming an individual skeletal muscle.

By "muscle cells", it is herein intended those cells that make or is directly involved in the structure of the muscle.
A muscle tissue comprises muscle cells (which build or are directly involved in the structure of the muscle tissue) and blood cells (from the circulating blood).
Therefore, the term "muscle cell" is not restricted to the meaning of "muscular fiber cells" or syncitial muscle cell; but blood cells are not encompassed by the term "muscle cells".
For example, skeletal muscle cells comprise blood-vessel forming cells, blood vessel-associated cells, motoneuron axon-associated cells, myelinating cells, extracellular matrix-producing cells, basal lamina-producing cells, stromal cells, endomysial cells, endothelial cells, angiogenic cells, myocytes, myogenic cells, syncitial muscle cells, but do not comprise blood cells.
The most preferred muscle cells of the invention are endomysial cells of the skeletal muscle.

Preferably, the muscle cells of said initial cell population comprise skeletal muscle cells and/or cardiac muscle cells, more preferably skeletal muscle cells, most preferably endomysial cells.

The muscle cells of said initial cell population may comprise the various muscle mononuclear cell sub-types that are comprised in the muscle tissue from which they originate or derive. Alternatively, they may comprise selected muscle mononuclear cell sub-type(s).

By cells of a single muscle cell sub-type, it is herein meant cells of a specific physiological sub-region or of specific sub-structure of a muscle.
More preferably, said specific sub-region is the sub-region of a skeletal muscle.
Specific sub-regions of a skeletal muscle notably comprise muscular fiber, basal lamina, endomysium, perimysium, epimysium.
Most preferably, said sub-region of skeletal muscle is the endomysium.

Therefore, the muscle cells of said initial cell population most preferably comprise cells from the endomysium of a skeletal muscle.

Preferably, prior to said ALDH+ cell enrichment, said initial cell population comprises a proportion of at least 40%, more preferably of at least 50%, even more preferably of at least 60%, still more preferably of at least 70%, even still more preferably of at least 80%, most preferably of at least 90% of said muscle cells.

Advantageously, the process of the present invention can be directly implemented, i.e., without any prior purification or enrichment, on an initial cell population that is as complex as the initial cell population, which is obtainable from a sample of muscle tissue that has been collected from a human or a non-human animal, e.g., from the initial cell population that is contained in a biopsy of skeletal muscle.
However, if desired, e.g., to further optimize the process yield, or if required by particular conditions, such as samples of poor quality, the initial cell population can be enriched in cells originating from the muscle (or depleted in some or all of those cells which do not originate from the muscle) prior to performing said enrichment in ALDH+ cells. This prior enrichment or depletion step may concern all the cells that are comprised in the sample, or only one or several specific muscle type(s) and/or sub-type(s) among those originally contained in the sample.

In the cell populations of the invention, as well as in the process of the invention, said muscle preferably is a mammalian muscle. Said mammal can be a human or a non-human animal.

When the cell populations of the invention are produced for medical applications to be performed on the human body or on a sample collected therefrom, said mammal preferably is a human.
When said cell population is produced for biotechnological applications, such as for the screening of molecules of biological and/or medical interest, said mammal can be a non-human mammal, at least for the first steps of said biotechnological application, e.g., for pre-screening steps.

Preferably, said human is a post-born human, such as a baby, an infant, a child, an adolescent, an adult.

Said non-human mammal can e.g., be a non-human primate, a rodent, an animal of genus *Sus*, an animal of the *Canis lupus* species, an animal of the *Ovis aries* species.
Preferably, said non-human primate is an animal of the *Cebidae* family such as the squirrel monkey (*Saimiri*), an animal of the *Cercopithecidae* family, such as a baboon (*Pan anubis*) or a macaque monkey (*Macaca mulatta*, *Macaca fascicularis*), a non-human animal of the *Hominidae* family such as a chimpanzee (*Pan troglodytes*).
Preferably, said rodent is an animal of the *Rodentia* order, such as a mouse (more particularly *Mus musculus*), a rat (more particularly *Rattus norvegicus*), a guinea pig, or an animal of the *Lagomorpha* order, such as a rabbit.
Preferably, said animal of the genus *Sus* is a pig.
Preferably, said animal of the *Canis lupus* species is a dog, more particularly a Golden Retriever Muscular Dystrophy dog.
Preferably, said animal of the *Ovis aries* species is a sheep
A preferred non-human mammal is a non-human primate, a mouse, a rabbit, a pig, a dog or a sheep.

A most preferred non-human mammal is a non-human primate.

Said human or non-human mammal muscle can be free of any muscle pathology or dysfunction, or, to the contrary, can have a muscle pathology or dysfunction. More generally, said human or non-human mammal can be free of any pathology or dysfunction that may affect a muscle, or, to the contrary, can have a pathology or dysfunction affecting a muscle, more particularly a striated muscle, still more particularly a skeletal muscle and/or a cardiac muscle and/or a smooth muscle.
Said human or non-human mammal muscle, which is free of any muscle affection, may further be *res nullus* from any muscle pathology or dysfunction and/or from any surgery that may affect or have affected a muscle, more particularly *res nullus* from any muscle and/or bone surgery.

Said human or non-human mammal muscle, which is free of any muscle pathology or dysfunction, may be a skeletal muscle, a cardiac muscle or a smooth muscle, preferably a skeletal muscle or a cardiac muscle, more preferably a skeletal muscle.

Said human or non-human mammal muscle may be a skeletal muscle, which has a pathology or dysfunction such as a pathology or dysfunction involving a muscular dystrophy, such as a myopathy, a Duchenne's muscular dystrophy, a Becker Muscular dystrophy, an Emery-Dreifuss muscular dystrophy, a limb-girdle muscular dystrophy, a facioscapulohumeral muscular dystrophy, an oculopharyngeal muscular dystrophy, a myotonic dystrophy, a congenital muscular dystrophy, an anal sphincter malformation or dysfunction, an urethral sphincter malformation or dysfunction, a muscle dysfunction caused by sport practice and/or trauma and/or accident, as well as a neuromuscular disorder, such as a spinal muscular atrophy, a lateral amyotrophic sclerosis, a Werdnig Hoffman disease, a Charcot-Marie tooth disease, a myositis.

Said human or non-human mammal muscle may be a cardiac muscle, which has a pathology or dysfunction such as an extrinsic cardiomyopathy (i.e., a cardiomyopathy, where the primary pathology is outside the myocardium itself) and/or an intrinsic cardiomyopathy (i.e., a weakness in the muscle of the heart that is not due to an identifiable external cause).

Extrinsic cardiomyopathy notably comprises coronary artery disease; some congenital heart diseases; nutritional diseases; ischemic cardiomyopathy; hypertensive cardiomyopathy; valvular cardiomyopathy; inflammatory cardiomyopathy; cardiomyopathy secondary to a systemic metabolic disease; alcoholic cardiomyopathy; diabetic cardiomyopathy.
Intrinsic cardiomyopathy notably comprises dilated cardiomyopathy, which is one of the leading indications for heart transplantation; hypertrophic cardiomyopathy, a genetic disorder caused by various mutations in genes encoding sarcomeric proteins; arrhythmogenic right ventricular cardiomyopathy; restrictive cardiomyopathy including the obliterative cardiomyopathy; noncompaction cardiomyopathy; some congenital heart diseases such as the Fallot's tetralogy.

Said human or non-human mammal muscle may be a smooth muscle, which has a pathology or dysfunction. Said pathology or dysfunction can be any pathology or dysfunction affecting a smooth muscle, but more particularly atherosclerosis, fibrosis, cirrhosis.

In the cell populations of the invention as well as in the cell populations produced by the process of the invention, said ALDH-positive cells may also be SSC^{lo} (low orthogonal light scattering). SSC is the 90° lights scatter, as assessed by flow cytometry. SSC^{lo} is preferably assessed under the flow cytometry settings that are described in example 1 below.

Said ALDH⁺-containing cell population of the invention notably has myogenic and/or adipogenic and/or osteogenic differentiation capacities. More particularly, contrary to the Side Population (SP) cells, the ALDH⁺-containing cell population of the invention has the capacity of undergoing myogenesis in the absence of co-cultured myoblasts or co-cultured cardiomyocytes.

Therefore the process of the invention more particularly is:
- a process for the production of a cell population suitable for myogenesis,
- a process for the production of a cell population suitable for adipogenesis,
- a process for the production of a cell population suitable for osteogenesis.

### Twelve additional features (CD45 feature; CD31 feature; ALP feature; myogenesis feature; CD133 feature; CD56 feature; CD90 feature; CD146 feature; two CD44 features; CD140b feature; CD105 feature):

As previously-mentioned and below-illustrated, said ALDH⁺-containing cell population of the invention comprises at least 50%, preferably at least 60%, more preferably at least 70%, still more preferably at least 80%, still even more preferably at least 90%, most preferably at least 95%, still most preferably at least 98%, even still most preferably at least 99% of ALDH-positive cells, for example 100% of ALDH-positive cells.
Therefore, said ALDH⁺-containing cell population of the invention mainly comprises ALDH-positive cells (at least 50% of ALDH-positive cells, as above-mentioned), but may nevertheless also contain some ALDH-negative cells (although at less than 50% of the total number of ALDH-positive and ALDH-negative cells that are contained in the cell population).
The ALDH-positive cells that are contained in an ALDH⁺-containing cell population of the invention may further show additional feature(s). Please see the examples below.

### CD45 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD45**-positive cells, for example no CD45-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD45 feature applies to those cells, which are ALDH-positive.

### CD31 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD31**-positive cells, for example no CD31-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD31 feature applies to those cells, which are ALDH-positive.

### Alkaline Phosphatase ("ALP") feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **ALP**-positive cells, for example no ALP-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said ALP feature applies to those cells, which are ALDH-positive.

### Myogenesis feature:

Preferably, said ALDH⁺-containing cell population of the invention is capable of undergoing **myogenic differentiation in the absence of co-cultured myoblasts or co-cultured cardiomyocytes.** Please see the examples below.

### CD133 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD133**-positive, cells, for example no CD133-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD133 feature applies to those cells, which are ALDH-positive.

### CD56 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD56**-positive cells, for example no CD56-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD56 feature applies to those cells, which are ALDH-positive.

### CD90-feature:

Preferably, the population of ALDH-positive cells that are contained in an ALDH⁺-containing cell population of the invention comprises **CD90**-positive cell(s), preferably 0.4-50%, more preferably 0.45-48%, still more preferably 0.5-45%, most preferably 0.5-40% of CD90-positive cells.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD90 feature applies to those cells, which are ALDH-positive.

### CD 146 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD146**-positive cells, for example no CD146-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD 146 feature applies to those cells, which are ALDH-positive.

### CD44 feature n° 1:

Preferably, the population of ALDH-positive cells that are contained in an ALDH⁺-containing cell population of the invention comprises **CD44-positive cells**, preferably 1-60%, more preferably 1.5-55%, most preferably 1.5-52% of CD44-positive cells.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD44 feature applies to those cells, which are ALDH-positive.

### CD44 feature n°2:

Preferably, the population of ALDH-positive cells that are contained in an ALDH⁺-containing cell population of the invention comprises no more than 5%, preferably no more than 4.5%, more preferably no more than 4%, still more preferably no more than 3%, even still more preferably no more than 2%, most preferably no more than 1% of **CD44**-positive cells, for example no CD44-positive cell.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD44 feature applies to those cells, which are ALDH-positive.

### CD140b feature:

Preferably, the population of ALDH-positive cells that are contained in an ALDH⁺-containing cell population of the invention comprises no more than 5%, preferably no more than 4.5%, more preferably no more than 4%, still more preferably no more than 3%, most preferably no more than 2% of **CD140b**-positive cells.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD 140b feature applies to those cells, which are ALDH-positive.

### CD105 feature:

Preferably, the population of ALDH-positive cells that are contained in an ALDH⁺-containing cell population of the invention comprises no more than 5%, preferably no more than 4.5%, more preferably no more than 4%, still more preferably no more than 3%, most preferably no more than 2% of **CD105**-positive cells.
These percentages are expressed with respect to the number of ALDH-positive cells that are contained in the ALDH⁺-containing cell population of the invention, i.e., without taking into account the number of ALDH-negative cells that may possibly be contained in an ALDH⁺-containing cell population of the invention.
Indeed, said CD105 feature applies to those cells, which are ALDH-positive.

In the present application, cells are considered to be CD133-negative and/or CD146-negative at least if they are appear to be CD133-negative and/or CD146-negative, respectively, when the treatment of the muscle tissue from which they derive or originate comprised an enzymatic collagenase and a trypsin treatment.

Means for the detection of the CD45, CD31, CD133, CD56, CD90, CD146, CD44, CD 140b, CD105 cell markers, means for the detection of the ALP enzymatic marker and means for the morphological study of myogenic differentiation are known to the person of ordinary skill in the art, e.g., by flow cytometry, cytochemistry, immunocytochemistry, immunocytofluorescence. Illustrative means are described in example 1 below.

Any combination of the twelve above-mentioned additional features is herein encompassed by the present application.

More preferably, said population of ALDH-positive cells that are contained in an ALDH⁺-containing cell population of the invention shows at least two, preferably at least three, more preferably at least four, still more preferably at least five, even still more preferably at least six, most preferably at least seven, still most preferably at least eight, even still most preferably at least nine, advantageously at least ten, very advantageously eleven of said twelve additional features.

Still more preferably, said population of ALDH-positive cells that are contained in an ALDH⁺-containing cell population of the invention:
- comprises no more than 5%, preferably no more than 4.5%, more preferably no more than 4%, still more preferably no more than 3%, even still more preferably no more than 2%, most preferably no more than 1% of **CD45**-positive cells, for example no CD45-positive cell; and
- comprises no more than 5%, preferably no more than 4.5%, more preferably no more than 4%, still more preferably no more than 3%, even still more preferably no more than 2%, most preferably no more than 1% of **CD31**-positive cells, for example no CD31-positive cell; and
- optionally shows at least one, preferably at least two, more preferably at least three, still more preferably at least four, even still more preferably at least five, most preferably at least six, still most preferably at least seven, even still most preferably at least eight, advantageously at least nine, very advantageously all of the ten other additional features (myogenesis, CD133, CD56, CD90, CD146, two CD44, CD140b, CD105 and ALP features).
   Among said ten other additional features, the CD133, the CD56 and the CD90 features are preferred; the CD133 and the CD56 features are more preferred features; and the CD133 feature is the most preferred feature.

Still more preferably, said population of ALDH-positive cells that are contained in an ALDH⁺-containing cell population of the invention:
- comprises no more than 5%, preferably no more than 4.5%, more preferably no more than 4%, still more preferably no more than 3%, even still more preferably no more than 2%, most preferably no more than 1% of **CD45**-positive cells, for example no CD45-positive cell; and
- comprises no more than 5%, preferably no more than 4.5%, more preferably no more than 4%, still more preferably no more than 3%, even still more preferably no more than 2%, most preferably no more than 1% of **ALP**-positive cells, for example no ALP-positive cell; and
- optionally shows at least one, preferably at least two, more preferably at least three, still more preferably at least four, even still more preferably at least five, most preferably at least six, still most preferably at least seven, even still most preferably at least eight, advantageously at least nine, very advantageously all of the ten other additional features (CD31, myogenesis, CD133, CD56, CD90, CD146, two CD44, CD140b, CD105 features).
   Among said ten other additional features, the CD133, the CD56 and the CD90 features are preferred; the CD133 and the CD56 features are more preferred features; and the CD133 feature is the most preferred feature.

Still more preferably, said population of ALDH-positive cells that are contained in an ALDH⁺-containing cell population of the invention:
- comprises no more than 5%, preferably no more than 4.5%, more preferably no more than 4%, still more preferably no more than 3%, even still more preferably no more than 2%, most preferably no more than 1% of **CD31**-positive cells, for example no CD31-positive cell; and
- comprises no more than 5%, preferably no more than 4.5%, more preferably no more than 4%, still more preferably no more than 3%, even still more preferably no more than 2%, most preferably no more than 1% of **ALP**-positive cells, for example no ALP-positive cell; and
- optionally shows at least one, preferably at least two, more preferably at least three, still more preferably at least four, even still more preferably at least five, most preferably at least six, still most preferably at least seven, even still most preferably at least eight, advantageously at least nine, very advantageously all of the ten other additional features (CD45, myogenesis, CD133, CD56, CD90, CD146, two CD44, CD140b and CD105 features).
   Among said ten other additional features, the CD133, the CD56 and the CD90 features are preferred; the CD133 and the CD56 features are more preferred features; and the CD133 feature is the most preferred feature.

Even still more preferably, said population of ALDH-positive cells that are contained in an ALDH⁺-containing cell population of the invention:
- is capable of undergoing **myogenic differentiation in the absence of co-cultured myoblasts or co-cultured cardiomyocytes;** and
- comprises no more than 5%, preferably no more than 4.5%, more preferably no more than 4%, still more preferably no more than 3%, even still more preferably no more than 2%, most preferably no more than 1% of **CD45**-positive cells, for example no CD45-positive cell; and
- comprises no more than 5%, preferably no more than 4.5%, more preferably no more than 4%, still more preferably no more than 3%, even still more preferably no more than 2%, most preferably no more than 1% of **CD31**-positive cells, for example no CD31-positive cell; and
- optionally shows at least one, preferably at least two, more preferably at least three, still more preferably at least four, even still more preferably at least five, most preferably at least six, still most preferably at least seven, even still most preferably at least eight, advantageously all of the nine other additional features (CD133, CD56, CD90, CD146, two CD44, CD140b, CD105 and ALP features).

Among said nine other additional features, the CD133, the CD56 and the CD90 features are preferred; the CD133 and the CD56 features are more preferred features; and the CD133 feature is the most preferred feature.

Even still more preferably, said population of ALDH-positive cells that are contained in an ALDH⁺-containing cell population of the invention:
- is capable of undergoing **myogenic differentiation in the absence of co-cultured myoblasts or co-cultured cardiomyocytes;** and
- comprises no more than 5%, preferably no more than 4.5%, more preferably no more than 4%, still more preferably no more than 3%, even still more preferably no more than 2%, most preferably no more than 1% of **CD45**-positive cells, for example no CD45-positive cell; and
- comprises no more than 5%, preferably no more than 4.5%, more preferably no more than 4%, still more preferably no more than 3%, even still more preferably no more than 2%, most preferably no more than 1% of **ALP**-positive cells, for example no ALP-positive cell; and
- optionally shows at least one, preferably at least two, more preferably at least three, still more preferably at least four, even still more preferably at least five, most preferably at least six, still most preferably at least seven, even still most preferably at least eight, advantageously all of the nine other additional features (CD31, CD133, CD56, CD90, CD 146, two CD44, CD 140b, CD105 features).
   Among said nine other additional features, the CD133, the CD56 and the CD90 features are preferred; the CD133 and the CD56 features are more preferred features; and the CD133 feature is the most preferred feature.

Even still more preferably, said population of ALDH-positive cells that are contained in an ALDH⁺-containing cell population of the invention:
- is capable of undergoing **myogenic differentiation in the absence of co-cultured myoblasts or co-cultured cardiomyocytes;** and
- comprises no more than 5%, preferably no more than 4.5%, more preferably no more than 4%, still more preferably no more than 3%, even still more preferably no more than 2%, most preferably no more than 1% of **CD31**-positive cells, for example no CD31-positive cell; and
- comprises no more than 5%, preferably no more than 4.5%, more preferably no more than 4%, still more preferably no more than 3%, even still more preferably no more than 2%, most preferably no more than 1% of **ALP**-positive cells, for example no ALP-positive cell; and
- optionally shows at least one, preferably at least two, more preferably at least three, still more preferably at least four, even still more preferably at least five, most preferably at least six, still most preferably at least seven, even still most preferably at least eight, advantageously all of the nine other additional features (CD45, CD133, CD56, CD90, CD 146, two CD44, CD 140b, CD105 features).
   Among said nine other additional features, the CD133, the CD56 and the CD90 features are preferred; the CD133 and the CD56 features are more preferred features; and the CD133 feature is the most preferred feature.

Most preferably, said population of ALDH-positive cells that are contained in an ALDH⁺-containing cell population of the invention shows all of said twelve features.

Preferably, notably with respect to the above-mentioned additional feature(s) and combination(s) of additional features, said population of ALDH-positive cells that are contained in an ALDH⁺-containing cell population of the invention represents at least 50%, preferably at least 60%, more preferably at least 70%, still more preferably at least 80%, still even more preferably at least 90%, most preferably at least 95%, still most preferably at least 98%, even still most preferably at least 99% of the total number of cells that are contained in the ALDH⁺-containing cell population of the invention (i.e., of the total of ALDH-positive cells and ALDH-negative cells).

Therefore the process of the invention may comprise as a step distinct or joint to said ALDH+ enrichment, one or several step(s) of depletion in those cells that are positive for one or several of said cell markers, as above described.

The invention also relates to a cell, which is obtainable by isolation from a cell population of the invention without any step of *in vitro* culture, more particularly without any step of *in vitro* proliferation and/or differentiation culture.

Said cell is ALDH-positive, and advantageously has at least two, preferably at least three, more preferably at least four, still more preferably at least five, even still more preferably at least six, most preferably at least seven, still most preferably at least eight, even still most preferably at least nine, advantageously at least ten, very advantageously eleven, most advantageously all of said twelve additional features.

In view of what precedes, the process of the invention may comprise as a step distinct or joint to said ALDH+ enrichment, one or several step(s) of enrichment in cells showing said feature(s).

### ALDH-positive and CD34-positive sub-populations, and ALDH-positive and CD34-negative sub-populations:

The inventors have further identified two distinct types of cell populations within said ALDH⁺-containing cell population of the invention, namely:
- a sub-population of ALDH-positive and CD34-negative cells;
- a sub-population of ALDH-positive and CD34-positive cells.
   The inventors have isolated each of these two sub-populations.

Said ALDH-positive and CD34-negative cell population notably has myogenic (as well as osteogenic) differentiation capacities. More particularly, contrary to the Side Population (SP) cells, this cell population has the capacity of undergoing myogenesis in the absence of co-cultured myoblasts or co-cultured cardiomyocytes.

Said ALDH-positive and CD34-positive cell population notably has adipogenic (as well as osteogenic) differentiation capacities.

Means for detecting the CD34 marker are available to the person of ordinary skill in the art, for example anti-CD34 antibodies, e.g., an APC-conjugated anti-CD34 antibody, such as the anti-CD34 antibody that is identified in Table 5 below.
Means for sorting cells in accordance with the CD34 cell marker are also available to the person of ordinary skill in the art, for example, by cytometry using an anti-CD34 antibody. Illustrative means for CD34 sorting are described in example 1 below.

Therefore, the present application further relates to:
- a cell population comprising ALDH-positive cells as herein defined and more particularly as above-defined, wherein said ALDH-positive cells comprise, more preferably are, CD34-negative cells, this population being herein referred to as an ALDH-positive CD34-negative cell population of the invention, and to
- a cell population comprising ALDH-positive cells as herein defined and more particularly as above-defined, wherein said ALDH-positive cells comprise, more preferably are, CD34-positive cells, this population being herein referred to as an ALDH-positive CD34-positive cell population of the invention.

Each of these cell populations may comprise at least 50%, preferably at least 60%, more preferably at least 70%, still more preferably at least 80%, still even more preferably at least 90%, most preferably at least 95%, still most preferably at least 98%, even still most preferably at least 99%, for example 100% of ALDH-positive and CD34-negative cells, or of ALDH-positive and CD34-positive cells, respectively.

Said ALDH-positive cells preferably comprise ALDH1-positive and/or ALDH3-positive cells, more preferably ALDH1-positive cells, most preferably ALDH1A1-positive cells.

As above-described, the population of ALDH-positive cells that are contained in an ALDH⁺-containing cell population of the invention may show one or several of said above-described (as well as below-illustrated) twelve additional features (CD45; CD31; ALP; myogenesis; CD133; CD56; CD90; CD146; - the two- CD44; CD140b; CD105 features). This(these) feature(s) and combination(s) of features may also apply to the ALDH-positive CD34-negative cell population of the invention and to the ALDH-positive CD34-positive cell population of the invention *mutatis mutandis.*
This is more particularly the case, when all the ALDH-positive cells that are contained in an ALDH-positive CD34-negative cell population of the invention are CD34-negative, and when all the ALDH-positive cells that are contained in an ALDH-positive CD34-positive cell population of the invention are CD34-positive, respectively.

Preferably, an ALDH-positive CD34-negative cell population of the invention does not comprise any ALDH-positive and CD34-positive cell, most preferably no CD34-positive cell. Preferably, an ALDH-positive CD34-positive cell population of the invention does not comprise any ALDH-positive and CD34-negative cell, most preferably no CD34-negative cell.

Preferably, the ALDH-positive CD34-negative cell population of the invention and the ALDH-positive CD34-positive cell population of the invention show one or several of ten of said above-described (as well as below-illustrated) additional features (CD45; CD31; ALP; myogenesis; CD133; CD56; CD90; CD146; -the two- CD44 features).

Preferably, said ALDH-positive CD34-negative cell population of the invention comprise no more than 2%, preferably no more than 1% of **CD140b**-positive cells, for example CD140b-positive cell.
Preferably, said ALDH-positive CD34-negative cell population of the invention shows the above-described (and below-illustrated) CD44 feature n°1, i.e., it comprises **CD44-positive cells,** preferably 1-60%, more preferably 1.5-55%, most preferably 1.5-52% of CD44-positive cells.

Preferably, said ALDH-positive CD34-positive cell population of the invention comprise no more than 2%, preferably no more than 1% of **CD105**-positive cells, for example no CD105-positive cell.
Preferably, said ALDH-positive CD34-positive cell population of the invention shows the above-described (and below-illustrated) CD44 feature n°2, i.e., it comprises no more than 5%, preferably no more than 4.5%, more preferably no more than 4%, still more preferably no more than 3%, even still more preferably no more than 2%, most preferably no more than 1% of **CD44**-positive cells, for example no CD44-positive cell.

More preferably, an ALDH-positive CD34-negative cell population of the invention:
- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD140b**-positive cells, more preferably no CD140b-positive cell, and/or comprises **CD44-positive cells,** preferably 1-60%, more preferably 1.5-55%, most preferably 1.5-52% of CD44-positive cells.
   More particularly, an ALDH-positive CD34-negative cell population of the invention:

- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD140b**-positive cells, for example no CD140b-positive cell.
   More particularly, an ALDH-positive CD34-negative cell population of the invention:

- shows one or several of eight of said above-described (as well as below-illustrated) additional features (CD45; CD31; ALP; myogenesis; CD133; CD56; CD90; CD146 features); and
- comprises **CD44-positive cells,** preferably 1-60%, more preferably 1.5-55%, most preferably 1.5-52% of CD44-positive cells.
   More particularly, an ALDH-positive CD34-negative cell population of the invention:

- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD140b**-positive cells, for example no CD140b-positive cell; and
- comprises **CD44-positive cells,** preferably 1-60%, more preferably 1.5-55%, most preferably 1.5-52% of CD44-positive cells.

More preferably, an ALDH-positive CD34-positive cell population of the invention:
- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD105**-positive cells, preferably no CD105-positive cell, and/or comprises no more than 5%, preferably no more than 4.5%, more preferably no more than 4%, still more preferably no more than 3%, even still more preferably no more than 2%, most preferably no more than 1% of **CD44-**positive cells, for example no CD44-positive cell.
   More particularly, the ALDH-positive CD34-positive cell population of the invention:

- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD105**-positive cells, for example no CD105-positive cell.
   More particularly, an ALDH-positive CD34-positive cell population of the invention:

- shows one or several of eight of said above-described (as well as below-illustrated) additional features (CD45; CD31; ALP; myogenesis; CD133; CD56; CD90; CD146 features); and
- comprises no more than 5%, preferably no more than 4.5%, more preferably no more than 4%, still more preferably no more than 3%, even still more preferably no more than 2%, most preferably no more than 1% of **CD44**-positive cells, for example no CD44-positive cell.
   More particularly, an ALDH-positive CD34-positive cell population of the invention:

- shows one or several of eight of said above-described (as well as below-illustrated) additional features (CD45; CD31; ALP; myogenesis; CD133; CD56; CD90; CD146 features); and
- comprises no more than 2%, preferably no more than 1% of **CD105**-positive cells, preferably no CD105-positive cell; and
- comprises no more than 5%, preferably no more than 4.5%, more preferably no more than 4%, still more preferably no more than 3%, even still more preferably no more than 2%, most preferably no more than 1% of **CD44**-positive cells, for example no CD44-positive cell.

The invention also relates to a cell, which is obtainable by isolation from a cell population of the invention without any step of *in vitro* culture, more particularly without any step of *in vitro* proliferation and/or differentiation culture.
Preferably, said cell is ALDH-positive and CD34-negative.
Advantageously, said ALDH-positive and CD34-negative cell further has at least one of, preferably at least two of, more preferably at least three of, even more preferably at least four of, still even more preferably at least five of, most preferably at least six or seven of, still most preferably all the following features: CD45-negative, CD31-negative, ALP-negative, CD133-negative, CD56-negative, CD140b-negative, CD105-negative, and the myogenesis feature as above-described.
Preferably, said ALDH-positive and CD34-negative cell is CD45-negative and CD31-negative and ALP-negative and CD133-negative.
More preferably, said cell, which is ALDH-positive, CD34-negative, CD45-negative, CD31-negative, ALP-negative and CD133-negative, further is CD56-negative.
Most preferably, said cell, which is ALDH-positive, CD34-negative, CD45-negative, CD31-negative, ALP-negative, CD133-negative and CD56-negative, further has said myogenesis feature.
Still most preferably, said cell, which is ALDH-positive, CD34-negative, CD45-negative, CD31-negative, ALP-negative, CD133-negative and CD56-negative, and which further has said myogenesis feature, further is CD 140b-negative.

Preferably, said cell is ALDH-positive and CD34-positive.
Advantageously, said ALDH-positive and CD34-positive cell further has at least one of, preferably at least two of, more preferably at least three of, even more preferably at least four of, still even more preferably at least five of, most preferably at least six of, still most preferably all the following features: CD45-negative, CD31-negative, ALP-negative, CD133-negative, CD56-negative, CD140b-negative, CD105-negative.
Preferably, said ALDH-positive and CD34-positive cell is CD45-negative and CD31-negative and ALP-negative and CD133-negative.
More preferably, said ALDH-positive, CD34-positive, CD45-negative, CD31-negative, ALP-negative and CD133-negative cell further is CD56-negative.
Most preferably, said ALDH-positive, CD34-positive, CD45-negative, CD31-negative, ALP-negative, CD133-negative and CD56-negative cell further is CD105-negative.

In view of what precedes, the process of the invention may comprise as a step distinct or joint to said ALDH+ enrichment, one or several step(s) of depletion or enrichment in CD34 cells, respectively.
Therefore, the process of the invention may further comprise enriching said muscle cell population in those of its cells that are CD34-negative and/or depleting said muscle cell population in those of its cells that are CD34-positive.
Such a process more particularly is a process for the production of a cell population suitable for myogenesis, or suitable as a pharmaceutical composition for the use in the treatment of muscle pathology or dysfunction.
Such a process more particularly is a process for the production of a cell population suitable for osteogenesis, or as a pharmaceutical composition for the use in the treatment of osteogenic pathology or dysfunction.

Alternatively, the process of the invention may further comprise enriching said muscle cell population in those of its cells that are CD34-positive and/or depleting said muscle cell population in those of its cells that are CD34-negative.
Such a process more particularly is a process for the production of a cell population that is suitable for adipogenesis, or suitable as a pharmaceutical composition for the use in the treatment of adipogenic pathology or dysfunction.
Such a process more particularly is a process for the production of a cell population suitable for osteogenesis, or as a pharmaceutical composition for the use in the treatment of osteogenic pathology or dysfunction.

Said CD34 enrichment or depletion step(s) may be distinct or joint to the ALDH enrichment step, as well as to any of the other enrichment or depletion steps that are herein described, more particularly with respect to said twelve additional cell features (CD45; CD31; ALP; myogenesis; CD133; CD56; CD90; CD146; - the two- CD44; CD140b; CD105 features).
The steps of a process of the invention may be performed in any order that the skilled person will find to be convenient or appropriate.
It is nevertheless advantageous to first proceed with the ALDH enrichment step.

As already mentioned above:
- said enrichment can be performed up to a certain cell proportion, e.g., up to a proportion at least 50% of the cells of said certain type in the enriched cell population;
- in the present application, the term "enriching" encompasses any enrichment proportion, but preferably any enrichment proportion of at least 50%, preferably at least 60%, more preferably at least 70%, still more preferably at least 80%, still even more preferably at least 90%, most preferably at least 95%, still most preferably at least 99% or more.
   More particularly, the enrichment in ALDH-positive CD34-negative cells or in ALDH-positive CD34-positive cells, respectively, is advantageously up to a proportion of at least 50%, preferably of at least 60%, more preferably of at least 70%, even more preferably of at least 80%, still more preferably of at least 90%, most preferably of at least 95%, still most preferably of at least 99% of said ALDH+ cells in the enriched cell population.

The present application also relates to the cell populations that are obtainable by implementation of a process of the invention on a muscle sample or biopsy, more particularly to the ALDH⁺-containing cell population of the invention, the ALDH-positive and CD34-negative cell population, the ALDH-positive and CD34-positive cell population that are obtainable by a process of the invention.

The features that are herein described equally apply to the cell populations, the cell(s) and to the process of the invention.

### Uses and applications:

As previously mentioned, the cell populations of the invention have differentiation capacities.

For example:
- said ALDH⁺-containing cell population of the invention notably has myogenic and/or adipogenic and/or osteogenic differentiation capacities;
- said ALDH-positive and CD34-negative cell population notably has myogenic and/or osteogenic differentiation capacities;
- said ALDH-positive and CD34-positive cell population notably has adipogenic and/or osteogenic differentiation capacities.
   An illustration of these progenic potentials is given in the examples below.

Therefore, the present application relates to each of the cell populations of the invention, for use as a cell population having differentiation capacities, more particularly for *in vitro or in vivo* use as a cell population having differentiation capacities.

More particularly, the present application relates to those cell populations of the invention, which comprise ALDH-positive cells as above described, for use as a cell population having myogenic differentiation capacities, more particularly for *in vitro or in vivo* use as a cell population in myogenesis.
More particularly, the present application relates to those cell populations of the invention, which comprise ALDH-positive cells as above described, for use as a cell population having adipogenic differentiation capacities, more particularly for *in vitro or in vivo* use as a cell population in adipogenesis.
More particularly, the present application relates to those cell populations of the invention, which comprise ALDH-positive cells as above described, for use as a cell population having osteogenic differentiation capacities, more particularly for *in vitro or in vivo* use as a cell population in osteogenesis.

More particularly, the present application relates to those cell populations of the invention, which comprise ALDH-positive and CD34-negative cells as above described, for use as a cell population having myogenic differentiation capacities, more particularly for *in vitro or in vivo* use as a cell population in myogenesis.
More particularly, the present application relates to those cell populations of the invention, which comprise ALDH-positive and CD34-negative cells as above described, for use as a cell population having osteogenic differentiation capacities, more particularly for *in vitro or in vivo* use as a cell population in osteogenesis.

More particularly, the present application relates to those cell populations of the invention, which comprise ALDH-positive and CD34-positive cells as above described, for use as a cell population having adipogenic differentiation capacities, more particularly for *in vitro or in vivo* use as a cell population in adipogenesis.
More particularly, the present application relates to those cell populations of the invention, which comprise ALDH-positive and CD34-positive cells as above described, for use as a cell population having osteogenic differentiation capacities, more particularly for *in vitro or in vivo* use as a cell population in osteogenesis.

In accordance with the present invention, each of the cell populations of the invention can be themselves administered to an animal.
This administration can be autologous or heterologous to the receiver human or non-human animal.
If desired or required, said administration may be preceded by the *in vitro* culture of said cell population or cells, for example to make cells proliferate and/or adhere.
Proliferation media are known to the person of ordinary skill in the art. Illustrative proliferation media are described in example 1 below; please see section "*Cell culture set up and expansion*". One of the preferred proliferation media comprises the modified MCDB medium, which is the MCDB120 medium, wherein L-valine has been replaced by D-valine, and wherein phenol red and thymidine have been omitted.
This modified MCDB medium has been described in WO 01/94555 and its US counter-part patent application(s) or patent(s) (e.g., US 2004 0043008 A1; US 2006 0088508 A1).
For example, the proliferation medium may consist of 80% modified MCDB medium, 20% Defined Fetal Bovine Serum (DFBS), Gentamicine (50µg/mL, Panpharma), 10 ng/mL human recombinant bFGF (R&D systems), 10⁻⁶ M dexamethasone (Merck).

Appropriate conditions of temperature and gas atmosphere are known and/or can be adjusted by the person of ordinary skill in the art.
Preferred conditions for proliferation of skeletal muscle cells are those which enable these cells to proliferate as adherent cells. Such conditions comprise the use of a culture medium that is appropriate to such a proliferation, such as the above-mentioned proliferation medium. Such conditions may additionally or alternatively comprise the use of temperature and/or gas atmosphere conditions which enable the cells to proliferate as adherent cells, for example a temperature of about 37°C and/or under a gas atmosphere at 5% CO₂.

Alternatively or additionally, each of the cell populations of the invention, as well as the cells of the invention can be placed *in vitro* under conditions inducing their differentiation, before being administered to a human or non-human animal.

Furthermore, each of the cell populations of the invention, as well as the cells of the invention, as well as any of the differentiated cells that are obtainable by inducing the differentiation of cells from a cell population of the invention or of cells of the invention, can be genetically engineered, for example for gene repair and/or replacement, and/or for gene addition and/or suppression, before being administered to the human or non-human animal and/or before being cultured for proliferation.

Those cell populations of the invention, which are suitable for use as a cell population or as cells having osteogenic differentiation capacities, can be used for the treatment of any bone and/or cartilage disorder.
Particular bone and/or cartilage disorders notably comprise osteoporosis, osteomyelitis, bone tumor (more particularly osteosarcoma); osteogenesis imperfecta; small and/or large bone defects; bone healing defects; more particularly osteoporosis, small and/or large bone defects, bone healing defects; still more particularly small and/or large bone defects, bone healing defects.
Particular cartilage disorders notably comprise osteoarthritis, achondroplasia, costochondritis, spinal disc herniation, relapsing polychondritis, cartilage tumor; more particularly osteoarthritis, spinal disc herniation, cartilage tumor.

Those cell populations of the invention, which are suitable for use as a cell population or as cells having myogenic differentiation capacities, can be used in the treatment of tumor or cancer.
For example, a cell population of the invention can be used to prevent the extension of a tumor and/or to deliver a biological and/or pharmaceutical agent to the tumor and/or to endothelial cells of the patient, more particularly to deliver an agent which is intended (or, in the case of an agent that is a nucleic acid, the expression product of which is intended) to have an anti-angiogenic effect such as thrombospondin-I, endostatin, angiostatin, platelet factor-4, interleukins, interferons, TNF-alpha, p53 and nucleic acids coding therefor.

Those cell populations of the invention, which are suitable for use as a cell population or as cells having myogenic differentiation capacities, can be used for the treatment of any disorder affecting a muscle, more particularly a skeletal muscle and/or a cardiac muscle and/or a smooth muscle, preferably a skeletal muscle and/or a cardiac muscle, more preferably a skeletal muscle, most preferably an endomysial tissue thereof.
Particular disorders affecting a skeletal muscle notably comprise any pathology or dysfunction involving a muscular dystrophy, such as a myopathy, a Duchenne's muscular dystrophy, a Becker Muscular dystrophy, an Emery-Dreifuss muscular dystrophy, a limb-girdle muscular dystrophy, a facioscapulohumeral muscular dystrophy, an oculopharyngeal muscular dystrophy, a myotonic dystrophy, a congenital muscular dystrophy, an anal sphincter malformation or dysfunction, an urethral sphincter malformation or dysfunction, a muscle dysfunction caused by sport practice and/or trauma and/or accident, as well as a neuromuscular disorder, such as a spinal muscular atrophy, a lateral amyotrophic sclerosis, a Werdnig Hoffman disease, a Charcot-Marie tooth disease, a myositis.

Particular disorders affecting a cardiac muscle notably comprise extrinsic cardiomyopathy and/or an intrinsic cardiomyopathy, including any disorder which may lead to heart transplantation.
Particular extrinsic cardiomyopathy notably comprises coronary artery disease, some congenital heart diseases, nutritional diseases affecting the heart, ischemic cardiomyopathy, hypertensive cardiomyopathy, valvular cardiomyopathy, inflammatory cardiomyopathy, cardiomyopathy secondary to a systemic metabolic disease, alcoholic cardiomyopathy, diabetic cardiomyopathy; preferably coronary artery disease, congenital heart disease, ischemic cardiomyopathy, hypertensive cardiomyopathy, valvular cardiomyopathy.
Particular intrinsic cardiomyopathy notably comprises dilated cardiomyopathy, hypertrophic cardiomyopathy, arrhythmogenic right ventricular cardiomyopathy, restrictive cardiomyopathy including the obliterative cardiomyopathy, noncompaction cardiomyopathy; preferably dilated cardiomyopathy; some congenital heart diseases, such as the Fallot's tetralogy.
Particular disorders affecting a smooth muscle notably comprise atherosclerosis, fibrosis; preferably, atherosclerosis.

Those cell populations, which are suitable for use as a cell population or as cells having adipogenic differentiation capacities, can be used for the treatment of any disorder affecting an adipose tissue.
Particular disorder affecting an adipose tissue notably comprise lipid disorder, including excess lipolysis, congenital lipodystrophy (or lipo-atrophic diabetes), Launois-Bensaude lipomatose, familial mesosomatic lipomatose, virus-related lipodystrophy, more particularly HIV-related lipodystrophy; preferably congenital lipodistrophy.

The term "treatment" is herein meant in its broadest meaning. It notably encompasses a preventive treatment and/or a palliative treatment and/or a curative treatment, including a tentatively curative treatment. Preferably, the term "treatment" means curative treatment, including a tentatively curative treatment.

The term "disorder" is herein meant in its broadest meaning. It notably encompasses a dysfunction and/or a disease.

The disorders to which the invention relates do not necessarily have their origin in the particular tissue, organ or body component that is affected.
For example, a muscular disorder may have its origin in the dysfunction of a muscle, or it may have its origin in a body component other than the muscle, while still affecting the muscle. Whether directly or indirectly affecting the muscle, both types of diseases, disorders and dysfunctions are encompassed by the present application.
The same applies to the bone(s) and/or cartilage diseases, disorders and dysfunctions, as well as to the adipose tissue diseases, disorders, dysfunctions to which the present application relates.

### Compositions:

In view of what precedes, the present invention more particularly relates to a composition, pharmaceutical composition, drug, cell or tissue therapy product, which comprises at least one cell population of the invention and optionally at least one pharmaceutically and/or physiologically acceptable vehicle.

Still more particularly, the present invention relates to any cell or tissue therapy product, which comprises at least one of the cell populations of the invention, which are suitable for use as a cell population having osteogenic differentiation capacities, and optionally at least one pharmaceutically and/or physiologically acceptable vehicle.
Still more particularly, the present invention relates to this cell or tissue therapy product, for use in the prevention, treatment, palliation of a bone and/or cartilage disorder, as herein, and more particularly as above-described.

Still more particularly, the present invention relates to any cell or tissue therapy product, which comprises at least one of the cell populations of the invention, which are suitable for use as a cell population having myogenic differentiation capacities, and optionally at least one pharmaceutically and/or physiologically acceptable vehicle.
Still more particularly, the present invention relates to this cell or tissue therapy product, for use in the prevention, treatment, palliation of a muscular disorder, as herein, and more particularly as above-described.

Still more particularly, the present invention relates to any cell or tissue therapy product, which comprises at least one of the cell populations of the invention, which are suitable for use as a cell population having adipogenic differentiation capacities, and optionally at least one pharmaceutically and/or physiologically acceptable vehicle.
Still more particularly, the present invention relates to this cell or tissue therapy product, for use in the prevention, treatment, palliation of an adipose tissue disorder, as herein, and more particularly as above-described.

The compositions, more particularly the therapy products of the present invention may further comprise at least one pharmaceutically and/or physiologically acceptable vehicle, such as at least one diluent, excipient, additive, pH adjuster, emulsifier or dispersing agent, pH buffering agents, preservative, surfactant, gelling agent, as well as buffering and other stabilizing and solubilizing agent, etc.

Appropriate pharmaceutically acceptable vehicles and formulations include all known pharmaceutically acceptable vehicles and formulations, such as those described in "Remington: The Science and Practice of Pharmacy", 20th edition, Mack Publishing Co.; and "Pharmaceutical Dosage Forms and Drug Delivery Systems", Ansel, Popovich and Allen Jr., Lippincott Williams and Wilkins.
In general, the nature of the vehicle will depend on the particular mode of administration being employed. For instance, parenteral formulations usually comprise, in addition to the one or more contrast agents, injectable fluids that include pharmaceutically and physiologically acceptable fluids, including water, physiological saline, balanced salt solutions, buffers, aqueous dextrose, glycerol, ethanol, sesame oil, combinations thereof, or the like as a vehicle. The medium also may contain conventional pharmaceutical adjunct materials such as, for example, pharmaceutically acceptable salts to adjust the osmotic pressure, buffers, preservatives and the like. The carrier and composition can be sterile, and the formulation suits the mode of administration.

Preferred vehicles are those, which are compatible with the survival of the cells of the invention in said composition or therapy product, such as albumin and/or plasma (preferably human plasma) and/or DMSO, preferably albumin and/or plasma (preferably human plasma).
Preferred vehicles also are those, which are compatible with a use in cell therapy, such as albumin and/or plasma (preferably human plasma).
Most preferred vehicles also are those, which are compatible with the survival of the cells of the invention and with a use in cell therapy, such as albumin and/or plasma (preferably human plasma).

A composition or therapy product of the invention can for example be a liquid solution, suspension, emulsion, or under the form of a frozen product. The composition can be formulated with traditional binders and carriers, such as triglycerides.

### Differentiation of the cells:

The present application further relates to a process, preferably an *in vitro* process, for the production of osteocytes and/or chondrocytes and/or bone(s) and/or cartilage, preferably of bone(s) and/or cartilage, which comprises producing at least one of the cell populations of the invention which have osteogenic differentiation capacities, and inducing the differentiation of said at least one cell population into osteocytes and/or chondrocytes and/or bone(s) and/or cartilage, preferably of bone(s) and/or cartilage, respectively.

The present application further relates to a process, preferably an *in vitro* process, for the production of myoblasts and/or myocytes and/or myotubes and/or muscle(s), preferably myocytes and/or myotubes and/or muscle(s), more preferably myotubes and/or muscle(s), which comprises producing at least one of the cell populations of the invention which have myogenic differentiation capacities, and inducing the differentiation of said at least one cell population into myoblasts and/or myocytes and/or myotubes and/or muscle(s), preferably myocytes and/or myotubes and/or muscle(s), more preferably myotubes and/or muscle(s), respectively.

The present application further relates to a process, preferably an *in vitro* process, for the production of preadipocytes and/or adipocytes and/or adipose tissue, preferably adipocytes and/or adipose tissue(s), most preferably adipose tissue(s), which comprises producing at least one of the cell populations of the invention having adipogenic differentiation capacities, and inducing the differentiation of said at least one cell population into preadipocytes and/or adipocytes and/or adipose tissue, preferably adipocytes and/or adipose tissue(s), most preferably adipose tissue(s), respectively.

Means for inducing the differentiation of a cell are available to the person of ordinary skill in the art. Such means usually involve culturing said cells onto or into a differentiation culture medium.
Illustrative differentiation culture medium for myogenesis notably comprises a medium containing DMEM (Gibco) supplemented with 10% Horse serum (AbCys).
Illustrative differentiation culture medium for adipogenesis notably comprises a medium containing DMEM (1 g/L glucose; Gibco) supplemented with 10% FBS, 0.5mM isobutyl-methylxanthine (Sigma), 60µM indomethacin (Sigma), 10⁻⁶M dexamethasone (Merck) and 5µg/mL insulin (Sigma).
Illustrative differentiation culture medium for osteogenesis notably comprises a medium containing DMEM (4.5 g/L glucose; Gibco) supplemented with 10% DFBS, 10⁻⁷M dexamethasone and 50µg/mL ascorbic acid.
Illustrative culture conditions are given in example 1 below.

Alternative culture medium and/or culture conditions can be found in the literature, e.g., in Pittenger *et al.* 1999.

The present application also relates to the differentiated cells that are obtainable by a process of the invention.

The present application also relates to a cell population of the invention, for use in the production of a differentiated cell or tissue therapy product.

Those cell populations of the invention, which are suitable for use as a cell population or as cells having myogenic differentiation capacities, can be made to differentiate into muscle progenitors and then into a muscle tissue. Such a muscle tissue can in turn be used in the treatment of tumor or cancer, e.g., as a vector for an anti-angiogenic agent.

More particularly, the present application relates to any one of the cell populations of the invention, which have myogenic differentiation capacities, for use as a cell population that is suitable for myogenesis, more particularly for use in the production of a differentiated cell or therapy product or of a tissue therapy product, wherein said therapy product is intended for the prevention, treatment, palliation of a muscular disorder.
For example, a cell population of the invention can be made to differentiate into a smooth muscle tissue; this smooth muscle tissue can in turn be used in the treatment of tumor or cancer. Said smooth muscle tissue may for example be used to prevent the extension of a tumor and/or to deliver a biological and/or pharmaceutical agent to the tumor and/or to endothelial cells of the patient, more particularly to deliver an agent which is intended (or, in the case of an agent that is a nucleic acid, the expression product of which is intended) to have an anti-angiogenic effect such as thrombospondin-I, endostatin, angiostatin, platelet factor-4, interleukins, interferons, TNF-alpha, p53 and nucleic acids coding therefor.
More particularly, the present application relates to any one of the cell populations of the invention, which have osteogenic differentiation capacities, for use as a cell population that is suitable for osteogenesis, more particularly for use in the production of a differentiated cell therapy product or of a tissue therapy product, wherein said therapy product is intended for the prevention, treatment, palliation of a bone and/or cartilage disorder.
More particularly, the present application relates to any one of the cell populations of the invention, which have adipogenic differentiation capacities, for use as a cell population that is suitable for adipogenesis, more particularly for use in the production of a differentiated cell therapy product or of a tissue therapy product, wherein said therapy product is intended for the prevention, treatment, palliation of an adipose tissue disorder.

The present application also relates to a process for preventing, treating, palliating a muscular disorder in an animal in need of such treatment, comprising administering to said animal at least one cell population of the invention that is suitable for myogenesis, preferably a physiologically acceptable amount thereof, optionally with at least one appropriate pharmaceutically and/or physiologically acceptable vehicle.
The present application also relates to a process for preventing, treating, palliating a muscular disorder in an animal in need of such treatment, comprising inducing the differentiation of a cell population of the invention that is suitable for myogenesis into myoblasts and/or myocytes and/or myotubes and/or muscle(s), and administering to said animal said differentiated cells or tissue(s), preferably a physiologically acceptable amount thereof, optionally with at least one appropriate pharmaceutically and/or physiologically acceptable vehicle.

The present application also relates to a process for preventing, treating, palliating a bone and/or cartilage disorder in an animal in need of such treatment, comprising administering to said animal at least one cell population of the invention that is suitable for osteogenesis, preferably a physiologically acceptable amount thereof, optionally with at least one appropriate pharmaceutically and/or physiologically acceptable vehicle.
The present application also relates to a process for preventing, treating, palliating a bone and/or cartilage disorder in an animal in need of such treatment, comprising inducing the differentiation of a cell population of the invention that is suitable for osteogenesis into osteocytes and/or chondrocytes and/or bone(s) and/or cartilage(s), and administering to said animal said differentiated cells or tissue(s), preferably a physiologically acceptable amount thereof, optionally with at least one appropriate pharmaceutically and/or physiologically acceptable vehicle.

The present application also relates to a process for preventing, treating, palliating a adipose tissue disorder in an animal in need of such treatment, comprising administering to said animal at least one cell population of the invention that is suitable for adipogenesis, preferably a physiologically acceptable amount thereof, optionally with at least one appropriate pharmaceutically and/or physiologically acceptable vehicle.
The present application also relates to a process for preventing, treating, palliating an adipose tissue disorder in an animal in need of such treatment, comprising inducing the differentiation of a cell population of the invention that is suitable for adipogenesis into preadipocytes and/or adipocytes and/or adipose tissue(s), and administering to said animal said differentiated cells or tissue(s), preferably a physiologically acceptable amount thereof, optionally with at least one appropriate pharmaceutically and/or physiologically acceptable vehicle.

The present application also relates to a process for the prognosis of the differentiation potential of cells that are contained in a muscle sample, preferably a muscle biopsy. The prognosis process of the invention comprises measuring the percentage of ALDH-positive and CD34-negative cells that are contained in the total number of ALDH-positive cells of said muscle sample, said percentage being termed Predictor Percentage, or "PP", by the inventors.

The inventors demonstrate that:
- it can be predicted that said muscle sample contains more cells having myogenic differentiation capacities than cells having adipogenic differentiation capacities, when said percentage (said PP) is of at least, or above the value of 25%; the accuracy of this prediction is of approximately 91 %;
- conversely, when said PP is below 25%, it can be predicted that said muscle sample contains more cells having adipogenic differentiation capacities than cells having myogenic differentiation capacities; the accuracy of this prediction is of approximately 70%;

The inventors further demonstrate that:
- when said PP is of at least, or above 40%, it can be predicted that said muscle sample contains more cells having myogenic differentiation capacities than cells having adipogenic differentiation capacities; the accuracy of this prediction is approximately of 100%.
   Please see example 1 below.

In view of what precedes, the present application also relates to the use, more particularly the *in vitro* use of the detection of ALDH (preferably of the detection of ALDH and of CD34), to predict the differentiation potential of cells that are (or were) contained in a muscle, e.g., in a muscle sample, such as a muscle biopsy.
Said detection may be performed on said muscle sample, for example after dissociation of the muscular fibers (e.g., after mechanical and/or enzymatic dissociation) so as to obtain a population of individual cells (individualized mononuclear cells) which were contained in said muscle sample.
Cells having an ALDH activity or wherein ALDH is present are indicative of osteogenic and/or myogenic and/or adipogenic differentiation capacities.
Said muscle(s) preferably is(are) skeletal muscle and/or cardiac muscle, preferably skeletal muscle endomysium and/or cardiac muscle, more preferably skeletal muscle endomysium.
Said use may comprise the detection of the presence or absence of ALDH and/or of an ALDH activity, and preferably the detection of the presence or absence of CD34 on said cells.
Said ALDH detection can be concomitant to said CD34 detection, or can be non-concomitant to said CD34 detection. For example, said ALDH detection may precede said CD34 detection.
In view of what precedes, said use preferably comprises the detection, and optionally the determination of the percentage of those cells that are ALDH-positive, preferably ALDH-positive and CD34-negative, as these cells are those which shows osteogenic and myogenic differentiation capacities, more particularly myogenic differentiation capacities.
In view of what precedes, said use preferably comprises the detection, and optionally the determination of the percentage of those cells that are ALDH-positive, preferably ALDH-positive and CD34-positive, as these cells are those which shows osteogenic and adipogenic differentiation capacities, more particularly adipogenic differentiation capacities.

The present application also relates to the use, more particularly the *in vitro* use of an ALDH substrate, more particularly of a fluorescent ALDH substrate, preferably of dipyrrometheneboron difluoxide aminoacetaldehyde (BAAA), for the detection of an ALDH activity, more particularly of an ALDH1 and/or ALDH3 activity, preferably an ALDH1 activity, more preferably an ALDH1A1 activity, on muscle cells, preferably on striated muscle cells (skeletal muscle cells and/or cardiac muscle cells), more preferably on skeletal muscle cells, still more preferably on the endomysial cells of the skeletal muscle.

Each of the relevant disclosures of all references cited herein is specifically incorporated by reference. The following examples are offered by way of illustration and not by way of limitation.

In an attempt to help the reader of the present application, the description has been separated in various paragraphs or sections. These separations should not be considered as disconnecting the substance of a paragraph or section from the substance of another paragraph or section. To the contrary, the present description encompasses all the combinations of the various sections, paragraphs and sentences that can be contemplated.

### EXAMPLES

### Example 1: aldehyde dehydrogenase (ALDH) activity identifies an endomysial population of human skeletal muscle cells with high myogenic capacities in vitro and in vivo [ALDH^{br}/SSC^{lo} cells, abbreviated as SMALD cells]

### Materials and methods

### Human muscle biopsies

Fresh and frozen human muscle biopsies were obtained for cell extraction and immunohistological analysis, respectively, in agreement with the French Regulatory Health Authorities and our Ethics committee via the Tissue Bank for Research of the French Association against Myopathies (Association Francaise contre les Myopathies; 1, rue de l'Internationale; BP 59; F - 91002 Evry Cedex; France). These were *res nullus* from orthopaedic surgery and consisted in 0.3-4 g specimen from *fascia lata* tensor or paravertebral muscles. The patients were free of muscular pathologies.

### Phenotypic characterization by immunohistochemistry

Seven micrometers cryostat sections of frozen human muscle biopsies were fixed in cold acetone (10 min, -20°C) and permeabilized with PBS containing 0.03% triton (10 min, Room Temperature -RT-). Non-specific labeling was blocked by incubation in PBS supplemented with 10% FBS (DFBS, Hyclone, 30 min, RT). Human ALDH was labeled using anti-ALHD mouse mAb (BD Pharmingen, 1/50, 4°C, overnight) followed by goat anti-mouse Ab (Alexa Fluor® 568, Molecular Probes, 1/1000, 1h, RT). Then, sections were incubated with a rabbit polyclonal anti-laminin Ab (Abcam, 1/200, 1h, RT), followed by a goat anti-rabbit Ab (Alexa Fluor® 488, Molecular Probes, 1/1000, 1h, RT) to delineate skeletal muscle fibers. Finally, the nuclei were labeled by DAPI contained in mounting medium (Vectashield® hard set + DAPI, Ab Cysteine). Negative controls were obtained by omitting primary Ab. Fluorescence imaging was assessed using Zeiss microscope and pictures captured using a Sony CCD cooled camera. The images were combined using Metamorph® software. All images were assembled in Adobe Photoshop®.

### Muscle processing

Fresh muscle fragments were minced and digested using collagenase (Liberase®; Roche, 1 h, 37°C), then trypsin-EDTA (0.25%, Hyclone-Perbio, 20 min, 37°C). The cell suspension was filtered through 100µm then 40 µm cell strainers (Becton-Dickinson) and pelleted. Cells were counted under haemacytometer, and either used directly or frozen for later use.

### Antibodies:

**Table 5: cellular markers**

| **CD / alternate name(s) / (function)** | **Clone #** | **Provider** | **Catalogue #** |
|---|---|---|---|
| CD10 / CALLA / (neutral endopeptidase) | W8E7 | BD | 347503 |
| CD 13 / gp 150 / (Aminopeptidase N) | L138 | BD | 347406 |
| CD15 / Lewis X, SSEA1 | MMA | BD | 332778 |
| CD31 / PECAM-1, Endo-CAM | WM59 | BD | 555446 |
| (PE-conjugated mAb) | | | |
| CD34 / gp105-120, Sialomucin | 8G12 | BD | 555822 |
| (APC-conjugated mAb) | | | |
| CD44 / Pgp-1, H-CAM | G44-26 | BD | 550989 |
| (PE-conjugated mAb) | | | |
| CD45 / LCA / (tyrosine phosphatase) | 2D1 | BD | 555483 |
| (PE-conjugated mAb) | | | |
| CD49e / Integrin α5β1 | IIA1 | BD | 555617 |
| CD56/N-CAM, Leu-19, NKH-1 | NCAM16-2 | BD | 345812 |
| (PE-conjugated mAb) | | | |
| CD73 / (Ecto 5' nucleotidase) | AD2 | BD | 550257 |
| CD90/Thy1 | 5E10 | BD | 555596 |
| (PE-conjugated mAb) | | | |
| CD105 / Endoglin | 1 G2 | BC | AO7414 |
| (PE-conjugated mAb) | | | |
| CD106 / VCAM-1 | 51-10C9 | BD | 555649 |
| (PE-conjugated mAb) | | | |
| CD117 / SCF receptor, c-kit / (tyrosine-kinase) | 104D2 | BD | 332785 |
| CD133 | AC133 | Miltenyi | 130-080-801 |
| (PE-conjugated mAb) | | | |
| CD140b | 28D4 | BD | 558821 |
| (PE-conjugated mAb) | | | |
| CD146 / MCAM, Mel-CAM, S-Endo-1, Muc18 | P1H12 | BD | 550315 |
| (PE-conjugated mAb) | | | |
| CD166 / ALCAM | 3A6 | BD | 559263 |
| Desmin / (Cytoskeleton filament) | D33 | Dako | MO760 |
| Lamin A-C / (Nuclear envelope) | 636 | Abcys | AbC10-L003 |
| Laminin / (Basal lamina component) | Polyclonal | Abcam | Ab1575-250 |
| α SMA / smooth muscle actin | 1A4 | Dako | MO851 |
| ALDH1 | 44 | BD | 611194 |

Antibodies are monoclonal, except as indicated. Most are IgG, except CD 15 (IgM).
BD: Becton-Dickinson
BC: Beckman Coulter -

### Flow cytometry analysis, phenotyping and sorting

Red cells were depleted from initial fresh bulk by incubation in lysis buffer (5 min, Beckman Coulter). Then cells were incubated in Aldefluor® assay buffer containing the ALDH substrate (Stem cell, 1µM, 20 min at 37°C). Control was obtained by prior incubation of cells with 50mM of the specific ALDH inhibitor diethylaminobenzaldehyde (DEAB). Extracellular markers were detected by incubations (1:20, 15 min, 4°C) with anti-CD34, anti-CD31, anti-CD44, anti-CD45, anti-CD56, anti-CD90, anti-CD105, anti-CD 106, anti-CD 140b, anti-CD 146, anti-CD 13 3 Abs (*cf*. table 5 above).

The cells were then centrifuged, suspended in Aldefluor® assay buffer. Fluorescence was detected using FL1 channel of flow cytometer (Facscalibur®, BD Pharmingen) and analysis performed using the Cell Quest® Software (10⁴ events analyzed). Populations were sorted using a Vantage SE DiVA® cell sorter on the basis of both ALDH activity and expression of CD34.

Instrument settings

**Cytometer Type: FACSCalibur BD Pharmingen**

| Detectors/Amps: | | | | |
|---|---|---|---|---|
| Param | Detector | Voltage | AmpGain | Mode |
| P1 | FSC | E00 | 1.00 | Lin |
| P2 | SSC | 281 | 5.02 | Lin |
| P3 | FL1 | 308 | 1.00 | Log |
| P4 | FL2 | 355 | 1.00 | Log |
| P5 | FL3 | 650 | 1.00 | Lin |
| P6 | FL2-A | | 1.00 | Lin |
| P7 | FL4 | 456 | | Log |

| Threshold: | | | | |
|---|---|---|---|---|
| Primary Parameter: FSC | | | | |
| Value: 253 | | | | |
| Secondary Parameter: None | | | | |

| Compensation: | | | | |
|---|---|---|---|---|
| FL1 - | 5.1 % | FL2 | | |
| FL2 - | 20.7 % | FL1 | | |
| FL2 - | 0.0 % | FL3 | | |
| FL3 - | 0.0 % | FL2 | | |
| FL3 - | 0.0 % | FL4 | | |
| FL4 - | 0.0 % | FL3 | | |

### Cell culture set up and expansion

Unsorted and sorted cells were seeded and expanded in the proliferation medium containing 80% modified custom-made MCDB medium, 20% Defined Fetal Bovine Serum (DFBS), Gentamicine (50µg/mL, Panpharma), 10 ng/mL human recombinant bFGF (R&D systems), 10⁻⁶ M dexamethasone (Merck).
The modified custom-made MCDB medium is the MCDB 120 medium (as described in Ham et al. 1988 "Improved media for normal human muscle satellite cells: serum-free clonal growth and enhanced growth with low serum" In vitro Cell Dev. Biol. 24: 833-844), wherein L-valine has been replaced by D-valine, and wherein phenol red and thymidine have been omitted. This modified custom-made MCDB medium has been described in WO 01/94555 and its US counter-part patent application(s) or patent(s) (e.g., US 2004 0043008 A1; US 2006 0088508 A1). Therefore, the modified custom-made MCDB medium is a serum-free medium, which is adapted to the growth of human muscle progenitor and/or stem cells, which does not comprise L-valine, phenol red and thymidine, but which comprises D-valine.
The medium was changed on the following day and replaced every 4 days. The cultures were settled for 7 to 14 days to reach 60% confluency, and then cells were harvested by trypsinization and expanded before reaching 80% confluency. Cells were grown for 2 or 3 passages and detached using trypsin-EDTA. An aliquot was labeled and analyzed as above for expression of CD56, CD34 and CD15 (BD Pharmingen). An aliquot was seeded for differentiation studies (see below). An aliquot was further expanded to assess the longitudinal fate of markers and ALDH expression in culture. In view of cell transplantation studies, a batch of myoblasts was prepared from unsorted cells as above.

### Cytofluorescence studies

Upon dissociation and sorting, fifty thousand SMALD/34⁺ or SMALD/34⁻ cells were seeded in 24-well dishes, and observed 18h or 6 days thereafter. Cells were incubated with the anti-CD 15, CD34, CD44, CD56, CD 146 Abs (*cf.* Table 5 above) at 1:20, 30 min, RT, respectively in culture medium, followed by goat anti-mouse Ab (Alexa Fluor® 568, 1:200, 20 min, RT). Cells were rinsed in substrate buffer and incubated in Aldefluor® assay buffer (20 min, 37°C) supplemented with DAPI (30µg/mL, Sigma), then rinsed in substrate buffer and observed immediately using an Olympus IX10 inverted microscope equipped for fluorescence analysis and a Sony CCD cooled camera. Images were captured and treated using the Metaview® software and paginated using Adobe Photoshop®.

### Alkaline phosphatase (ALP)

Flow cytometry sorted cells were centrifuged on glass slides for ten minutes and were assayed for ALP activity by incubation (10 min) with 5-bromo-4-chloro-3-indolylphosphate/nitro blue tetrazolium (BCIP/NBT) buffered substrate (Sigma).

### In vitro differentiation assays

*Myogenesis*. Fifty thousand cells were seeded into 12-wells plates. Differentiation was induced 24 hours later with a medium containing DMEM (Gibco) supplemented with 10% Horse serum (AbCys). Four to five days later, cells were permeabilized and fixed by cold methanol (10 min, -20°C). The expression of fast MyHC in differentiated myotubes was assessed using anti-fast MyHC mAb (clone MY32, Sigma, 1/400, 1 h, RT) followed by a goat anti-mouse IgG (H+L) Ab (Alexa Fluor® 568, 1/1000, 1 h, RT). The nuclei were labeled with DAPI contained in mounting medium (Vectashield® + DAPI, AbCys).
*Adipogenesis*. Hundred thousand cells were seeded into 6 well plates. Differentiation was induced 48 hours later with the adipogenic medium containing DMEM (1 g/L glucose; Gibco) supplemented with 10% FBS, 0.5mM isobutyl-methylxanthine (Sigma), 60µM indomethacin (Sigma), 10⁻⁶M dexamethasone (Merck) and 5µg/mL insulin (Sigma). The medium was replaced every 3-4 days for 21 days. The cells containing lipid vacuoles were photographed under phase contrast.
*Osteogenesis*. Hundred thousand cells were seeded into 6-well plates. Differentiation was induced 48 hours later with the osteogenic medium containing DMEM (4.5 g/L glucose; Gibco) supplemented with 10% DFBS, 10⁻⁷M dexamethasone and 50µg/mL ascorbic acid.
To enable mineralization process, 3mM NaH₂PO₄ were added to the wells reserved for alizarine staining. Medium was replaced every 3-4 days for 14 days. Then, cultures were washed with PBS, fixed in a solution of cold methanol (10 min, -20°C) and stained for ALP activity or Alizarine red. To observe ALP activity, cells were incubated 10 min with 5-bromo-4-chloro-3-indolyl phosphate / nitro blue tetrazolium (BCIP/NBT) buffered substrate (Sigma). To observe the mineralization process, fixed cells were stained for 10 minutes with Alizarin red solution (pH 4.1; Sigma).

### In vivo muscle pretreatment and cell transplantation

All the procedures were conducted according to the Guide for the Care and Use of Laboratory animals (DHAW publication n°85-23 Office of Science and Health Reports, DRR/NIH, Bethesda MD 20892). All procedures were performed under anesthesia (80 mg/kg Ketamine, 16 mg/kg xylazine). *Tibialis anterior* muscles of two-month-old female SCID mice (Charles Rivers) were pretreated by 18Gy cobalt irradiation, followed by the injection of 5ng of notexin (Sigma), respectively 2 to 4 days and one day before cell implantation. Freshly sorted human cells or long-term myoblast culture at third passage were counted, suspended in PBS containing 0.5% BSA (Sigma) and kept on ice until transplantation in 8-10 sites under 10 µl using a custom-made glass needle. Both legs received the same cell population. The numbers of injected cells are indicated in Table 2 below (in the "Results" section).

### Analysis and quantification of cell implantation in vivo

The animals were killed 4 weeks after transplantation. Injected muscles were snap-frozen in nitrogen-cooled isopentan and 7µm cryostat sections performed from tendon to tendon in order to ensure a complete overview of human cell distribution in muscle.
The presence of human cells in host muscle and their participation to muscular regeneration were evaluated by species-specific labeling of the human proteins lamin A/C and COX2, while laminin staining defined the basement membrane localization. Muscle sections were permeabilized with PBS containing 0.03% triton (10 min, RT) and non-specific labeling was blocked by incubation in PBS supplemented with 10% FBS (30 min, RT). Then, sections were incubated with rabbit polyclonal anti-COX2 Ab (1/1000, 1h, RT, obtainable by immunizing rabbits against COX2 as described in Lombes *et al.* 1989) and IgG2b anti-human lamin A/C mAb (1/100, 1h, RT, AbCys), followed by goat anti-rabbit IgG (H+L) Ab (Alexa Fluor® 488, 1/1000, 1h, RT) and goat anti-mouse IgG2b Ab (Alexa Fluor® 568, 1/1000, 1h, RT). Then, sections were incubated with rabbit polyclonal anti-laminin Ab (1/200, 1h, RT) followed by goat anti-rabbit IgG (H+L) Ab (Alexa Fluor® 350, 1/1000, 1h, RT). Slides were mounted in mowiol. Negative controls were obtained by omitting primary Ab. Image captures were performed as above.

Eight to ten representative sections were selected at regular intervals from tendon to tendon for each cell transplantation condition, to quantify the numbers of human nuclei located outside or inside muscle fibers. Images of lamin A/C and laminin labeling were overlapped (25X magnification) and all human nuclei counted. Then inferred number of human nuclei (IN), percentage of nuclei included in muscular fibers (% NIF), and ratio of inferred number of human nuclei on number of injected cells (RNC) were calculated as described below:
IN = (number of nuclei X muscle length) / (number of sections X section width)
% NIF = [(total number of nuclei) / (number of nuclei inside fibers)] X 100
RNC = (IN) / (number of injected cells)

### Statistical analyses

ANOVA test documented significance of differences between samples as indicated in the tables and figures.
The (predictor %) PP threshold value enabling to predict culture phenotype of one biopsy was determined by statistical analysis. After ensuring that values of both groups [56+>15+] and [56<15+] were distributed according normal laws, the inventors performed extrapolation of these distributions and determined the PP threshold value when both groups [56+>15+] and [56+<15+] presented equal probability according to Bayes law.

### Results

### Histological and flow cytometry characterization of ALDH-positive cells in human skeletal muscle

Immunohistofluorescence analysis of human skeletal muscle biopsies sections using an anti-ALDH1 Ab highlighted rare cells, i.e. less than 2 per 25mm² section, presenting a cytoplasmic expression of ALDH (Figure 1A, green). The presence of a nucleus was ascertained by DAPI co-staining (Figure 1A, blue). The delineation of the muscle fibers by laminin staining (Figure 1A, red) indicated that cells containing ALDH1 were located in the endomysial compartment (Figure 1A, merge), as opposed to the classical localization of satellite cells underneath the muscle fiber basal lamina. ALDH activity was classically measured using the commercial reagent Aldefluor®. Being a fluorescent substrate of the enzyme, it accumulates inside the cells upon its oxidation by the enzyme and is detected by the green fluorescence (FL1) channel of a standard flow cytometer. ALDH activity was analyzed in mononucleated cell suspensions dissociated from human muscle biopsies (n = 12) in specific conditions described above. Negative controls were obtained by analysis of samples pre-incubated with DEAB, an inhibitor of ALDH activity. A small population of cells with low side-scatter (SSC^{lo}) and bright ALDH (ALDH^{br}) activity presented a shift of fluorescence in absence (Figure 1B) but not in presence of DEAB. These characteristics were similar to that of human cells from bone marrow, umbilical cord blood, peripheral blood, and of murine cells from neural tissue. The median percentage of this skeletal muscle ALDH^{br}/SSC^{lo} population (abbreviated SMALD) represented approximately 3-4% of the bulk mononucleated muscle cell suspension (Figure 1B, gate 2). Similar results were obtained using fresh or frozen and thawed cell suspensions.

### Phenotypic characterization of SMALD populations

Aldefluor® measurement was first coupled with CD34 labeling, which was repeatedly described as a co-marker of human cells with high ALDH activity.

This co-labeling clearly highlighted two very distinctly separated sub-populations of SMALD cells: double positive ALDH⁺/34⁺ (SMALD/34⁺) cells (Figure 1B, gate 3) and simple positive ALDH⁺/34⁻ (SMALD/34⁻) cells (Figure 1B, gate 4). A third muscle cell population was defined as simply positive for CD34 (ALDH⁻ CD34⁺ cells, also herein referred to as "spCD34") and represented a median percentage of 8.0% of mononucleated cells. Finally, negative cells for both ALDH activity and CD34 marker (double negative, DN) represented the main population contained in a dissociated muscle biopsy as they reached the median percentage of 76%.
The phenotypes of these four subpopulations were then further characterized by flow cytometry by mean of triple labeling (Figure 1C and Table 1).

**Table 1: Flow cytometry phenotypic characterization of dissociated muscle cells**

| **Initial biopsy population** | **ALDH+ CD34-** (SMALD/34-) | **ALDH+ CD34+** (SMALD/34+) | **ALDH- CD34+** (spCD34) | **ALDH- CD34-** (DN) |
|---|---|---|---|---|
| **CD31+** | **0%** | **<1%** | 26.9% | <1% |
| n=3 | | | (5.1-55.5) | |
| CD44+ | 19.6% | 1.0% | <1% | <1% |
| n=4 | (0.0-51.0) | (0.0-1.5) | | |
| CD45+ | 0% | 0% | <1% | 2.7% |
| n=3 | | | | (0.7-4.2) |
| **CD56+** | **0%** | **0%** | 1.4% | 2.8% |
| n=12 | | | (0.0-21.5) | (0.4-43.4) |
| CD90+ | 5.9% | 6.6% | 5.3% | 2.6% |
| n=5 | (1.2-8.5) | (0.7-39.0) | (3.5-16.9) | (1.8-21.8) |
| **CD105+** | 1.5% | 0% | 7.4% | 1.4% |
| n=4 | (0.0-2.6) | | (2.0-27.7) | (0.1-3.1) |
| **CD133+(*)** n=4 | **0%** | **0%** | <1% | 0% |
| **CD140b+** | **0%** | 1.6% | 0% | <1% |
| n=3 | | (0.2-1.6) | | |
| **CD146+(*)** n=3 | **<1%** | **0%** | <1% | 0% |

| | | | | |
|---|---|---|---|---|
| (*) CD146 and CD133 expression as detected after dissociation of the muscle fibers by enzymatic treatment [collagenase type 1 and trypsin-EDTA treatment]. | | | | |

The percentages indicated in Table 1 are median percentages of cells positive for the marker in the given population. For each marker, higher and lower values are indicated between brackets.

A median percentage value of 0% or of <1% correspond to values that are lower than the detection limits.

Aldefluor® activity, CD34 expression, and the presence of several cell surface antigens were detected using the green (FL1), blue (FL4) and red (FL2) fluorescence channels, respectively. The inventors specifically analyzed, one by one, the expression of markers of committed progenitors: CD31 (PECAM-1) for endothelial cells, CD44 (Pgp-1), CD90 (Thy-1), CD105 (TGF-beta1/3-R) for mesenchymal cells, CD45 and CD133 for hematopoietic cells, CD56 (NCAM) for myogenic cells, CD140b (PDGF-Receptor beta) and CD146 (S-endo-1) for pericytes (while overlaps exist between the functional roles of these markers).
CD90 was found expressed in close percentages in the four populations, and may not be a discriminant marker (Table 1). The other ones, however, often exhibit specific enrichment according to each population considered.
SMALD/34⁻ population contained a small percentage of cells expressing CD90 and CD105 antigens, and a higher proportion of cells expressing CD44, although with important inter-individual variations. SMALD/34⁻ population was found negative for other markers: CD31, CD45, CD56, CD133 and CD140b, while very few cells expressed CD 146, if any (less than 1 %) (Table 1).
SMALD/34⁺ population contained small percentages of cells positive for CD44, CD90, CD140b, but was negative for CD45, CD56, CD105, CD133, CD146 and CD31. Taken together, these data demonstrate that SMALD cells were not a population of committed progenitors of endothelial, mesenchymal, hematopoietic or myogenic lineages, nor pericytic-like cells, but a population, which essentially comprises primitive progenitors. Overall, CD44 would be cautiously informative and/or discriminant for the SMALD/34⁻ population.
Single positive CD34⁺ population ("spCD34") contained variable percentages of cells expressing the tested markers, but this population was especially enriched in CD31⁺ cells, thus suggesting a pro-endothelial commitment of a significant fraction of the population, further underlined by the expression of CD105. CD44, CD45, CD133 and CD146 expression were detected on very low percentages of these cells (less than 1%), while CD140b was found completely negative. Interestingly, CD56⁺ cells were present in this population, suggesting that a small percentage of the cells expressed commitment markers of myogenesis and angiogenesis, as described for myo-endothelial cells. Overall, CD31 would be informative and relatively discriminant for the spCD34 population (Table 1).

DN population contained cells positive for CD45, CD56, CD90 and CD105, very small percentages of CD31⁺, CD44⁺, CD140b⁺ cells, and was completely negative for CD133 and CD146. This DN population included the most important percentage of CD56⁺ cells, reflecting the myogenic commitment that is later revealed in culture under myogenic conditions. Interestingly, mature circulating hematopoietic progenitors expressing CD45 are gathered in this DN fraction, while they are not observed in the other ones (Table 1).

### Phenotype and growth of cell-sorted populations

Freshly dissociated or frozen and thawed cells were sorted upon their expression of ALDH activity and expression of CD34. The four populations (SMALD/34⁺, SMALD/34⁻, spCd34 and DN) were collected separately using a FACS Vantage SE Diva (Figure 2A), gaining a purity above 95%. The indicated purity of selection was confirmed by re-analysis of each sorted population. The selected populations were plated in myogenic proliferation medium, concurrently to a non-sorted (NS) fraction used as a quality control of muscle cell culture. The spCD34 cells remained quiescent and rapidly degenerated with time in this proliferation medium. The other cell populations adhered to the substrate, proliferated (Figure 3, column A) and were harvested on passages 2-3 first for flow cytometry phenotypic analyses of ALDH activity and expression of CD34, CD56, CD15, and second for differentiation assays *in vitro.*
As observed in NS population, the CD34 marker was not conserved in any sorted population in culture, although this was a specific marker of the SMALD/34⁺ cells at initial dissociation.
The percentages of CD56⁺ and CD15⁺ cells (Figure 3E) in NS cells raised 76% and 16%, respectively, with high standard deviations reaching 28% and 23%, respectively. These deviations underline the inter-sample variations that could be attributed to differences in the anatomical origin of the biopsies. Interestingly, 34⁻ and 34⁺ SMALD-derived cells presented specific differences, whatever the biopsy. Indeed SMALD/34⁻-derived cell cultures contained on average 94+/-6% of CD56⁺ cells and never expressed CD15, and appeared to be a homogenous population of myoblasts. On the opposite, SMALD/34⁺-derived cells never presented a CD56⁺ phenotype, but 44+/-19% of them expressed CD15, and were therefore considered as a heterogeneous population of CD56⁻ cells. DN-derived cells contained 51+/-41% CD56⁺ cells. The DN fraction is heterogeneous and initially contained CD56⁺ cells, which indeed expanded in the myogenic proliferation medium. Similarly, DN-derived cells contained 17+/-19% CD15⁺ cells.
Taken together, these results suggest a double (at least) origin for the CD56⁺ cells obtained in course of the cell culture process, with one emerging from the SMALD/34⁻ fraction, and the other one emerging from the initial pool of CD56⁺ satellite cells. Similarly, CD15⁺ cells would emerge simultaneously from the SMALD/34⁺ and the DN fraction without a specifically identified marker.

Immunocytofluorescence analysis of SMALD/34⁻ and SMALD/34⁺ cells *in vitro* confirmed and illustrated the flow cytometry data (Figure 2B). SMALD/34' cells were found positive only for CD44 within hours after seeding and negative for CD34, CD56, CD15, and they expressed CD44, CD56 and/or CD146 in culture. Conversely, SMALD/34⁺ cells expressed CD34 immediately after seeding, but this marker was lost in culture, while CD15 and/or CD44 appeared within 6 days (Figure 2B), and these cells remained negative for CD56.
The SMALD/34⁺ and the SMALD/34⁻ cells were ALP-negative (*cf*. Figure 10).

### Differentiation of cell-sorted populations in vitro

After 4-5 days in myogenic differentiation medium, SMALD/34'-derived cells fused and formed multinucleated myotubes expressing the fast myosin isoform. Placed in the same conditions, SMALD/34⁺-derived cells were unable to fuse into myotubes and consequently stained negative for fast myosin (Figure 3, columns B, C). However, these SMALD/34⁺-derived cells differentiated extensively in adipoblast-like cells filled with numerous lipidic vesicles (Figure 3, Column D). On opposite, SMALD/34⁻-derived cells fused, differentiated into multinucleated myotubes in this medium (Figure 3, Columns B, C), and never presented this adipogenic phenotype *in vitro* (Figure 3, Column D).
Osteogenic differentiation was assessed *in vitro* by positive alkaline phosphatase (ALP) cytoenzymatic staining (blue) and alizarin staining of mineralization process (red). After two weeks in osteogenic medium, SMALD/34'- and SMALD/34⁺- derived cells presented similar positive staining for both markers. However, at cellular level, SMALD/34'-derived cells consisted of multiple myotubes stained positively for ALP (Figure 7).
Taken together, our results suggest that SMALD/34⁻ and SMALD/34⁺ cells progressed in culture along two different pathways associated to myogenic or mesenchymal-like potentials, respectively.

### Prognostic value of SMALD/34⁻ versus total SMALD cells in culture

Dissociated muscle cells from 20 biopsies were both initially phenotyped on ALDH and CD34 expression, and seeded in myogenic proliferation medium. After 2 to 3 passages, the phenotypes of cultured cells were analyzed by flow cytometry for CD56 and CD15 markers, and data were distributed into two groups according to the final yield in CD56⁺ and CD15⁺ cells. A first group of 9 biopsies comprised the events where the number of CD56⁺ cells was superior to that of CD15⁺ cells. The second group included 11 biopsies from which the yield of CD15⁺ cells was higher than that of CD56⁺ cells (Figure 4). The inventors calculated and compared the initial percentages of SMALD/34⁻ cells contained in the total number of SMALD cells in both groups, which were termed predictor percentages (PP). The PP presented farther median distribution values (33.8 % for the first group and 13.8% for the second group) which were significantly different between groups (p < 0.01). Using Bayes law, the inventors calculated that a PP threshold value fixed at 25% allowed discriminating the two groups. Below the 25% threshold value, a given biopsy would provide a cell culture containing less CD56⁺ cells than CD 15⁺ cells, with an accuracy of 73%. Above the 25% threshold value, a given biopsy would provide a cell culture containing more CD56⁺ cells than CD15⁺ cells, with an accuracy of 91%. If PP threshold is fixed at 40%, the accuracy for obtaining more CD56⁺ than CD15⁺ cells raises 100%. In view of clinical or industrial applications, since human muscle biopsies are rare and precious, it is important to evaluate the percentage of biopsies potentially excluded by the predictor test. When PP threshold value is fixed at 25% to delineate the groups, 8% of biopsies with PP superior to 25% would yield a majority of CD15⁺ cells, while on the other side 27% of biopsies with PP inferior to 25% would yield a majority of CD56⁺ cells. Overall, the analysis of ALDH activity and concomitant CD34 labeling provided predictive indication on the evolution of cell culture issued from human muscle tissue (Figure 4). Further studies are mandated to rely this prognostic feature to the anatomical origin of muscle, or donor parameters.

### In vivo differentiation abilities of sorted populations

The model selected for implantation required 2 steps of pretreatments. First, irradiation of the mouse leg eliminated the intrinsic regenerative ability of the host cells and conferred a repopulating advantage to donor cells. Second, the induction of acute degeneration by the snake venom notexin initiated intense muscle remodeling, with consequent activation of the repair mechanisms. In the present study, several muscle biopsies were pooled together to warrant the extraction of sufficient numbers of cells (i.e., at least 5,000) for subsequent implantation, and therefore the final number of animal data remained low (2-3 for each cell grafting condition). Nevertheless, the results were extremely consistent for each considered population, as presented (*cf*. Table 2 below).

In table 2, each line describes the results observed or calculated from one *tibialis* anterior muscle. The total number of human nuclei was counted on 8 sections covering the entire length of the muscle and estimated number was calculated as described.
**IN:** inferred number of human nuclei.
IN = (number of nuclei X muscle length) / (number of sections X section width)
**%NIF:** percentage of nuclei included in muscular fibers.
% NIF = [(total number of nuclei) / (number of nuclei inside fibers)] X 100
**RNC:** ratio of inferred number of human nuclei on number of injected cells.
RNC = (IN) / (number of injected cells)

**Table 2: Implantation and regeneration efficiencies of different muscle cell populations in vivo.**

| **Type of injected cells** | **Number of injected cells** | **IN** | **% NIF** | **RNC** | **n** |
|---|---|---|---|---|---|
| **Myoblasts** | 5,000 | 3,796 | 74 | 0.76 | |
| | 5,000 | 1,776 | 79 | 0.36 | |
| | 5,000 | 2,755 | 60 | 0.55 | |
| Mean +/- SD | 5,000 | 2,776+/-1,010 | 71+/-10 | 0.56 +/- 0.20 | 3 |
| Myoblasts | 20,000 | 18,000 | 60 | 0.90 | |
| | 20,000 | 29,449 | 63 | 1.47 | |
| | 20,000 | 45,429 | 63 | 2.27 | |
| Mean +/- SD | 20,000 | 30,959 +/- 13,777 | 62 +/- 2 | 1.55 +/- 0.69 | 3 |
| **SMALD/34-** | 6,000 | 33,429 | 52 | 5.57 | |
| | 20,000 | 40,667 | 58 | 2.03 | |
| Mean +/- SD | 13,000 +/- 9,900 | 37,048 +/- 5,118 | 55 +/- 4.2 | 3.80 +/- 2.50 | 2 |
| **SMALD/34+** | 12,000 | 1,018 | 0 | 0.08 | |
| | 12,000 | 1,018 | 5 | 0.08 | |
| | 20,000 | 696 | 0 | 0.03 | |
| | 22,000 | 1,554 | 3 | 0.07 | |
| | 22,000 | 2,946 | 0 | 0.13 | |
| Mean +/- SD | 17,600 +/- 5,200 | 1,446 +/- 893 | 1.6+/-2.3 | 0.08 +/- 0.03 | 5 |
| **Double Negative** | 20,000 | 0 | 0 | 0 | |
| | 20,000 | 0 | 0 | 0 | |
| | 20,000 | 0 | 0 | 0 | |
| Mean +/- SD | 20,000 | 0 +/- 0 | 0 +/- 0 | 0 +/- 0 | 3 |
| Double negative | 300,000 | 5,449 | 57 | 0.02 | |
| | 300,000 | 161 | 0 | 0.01 | |
| | 300,000 | 1,661 | 19 | 0.01 | |
| Mean +/- SD | 300,000 | 2,424 +/- 2,725 | 25 +/- 29 | 0.01 +/- 0.01 | 3 |
| **Non sorted** | 10,000 | 964 | 22 | 0.1 | |
| | 20,000 | 3,536 | 38 | 0.18 | |
| Mean +/- SD | 15,000 +/- 7,700 | 2,250+/-1,818 | 30+/-11.3 | 0.14 +/- 0.05 | 2 |
| Non sorted Non sorted | 80,000 100,000 | 2,732 4,179 | 45 58 | 0.03 0.04 | |
| Mean +/- SD | 90,000 +/- 14,100 | 3,455 +/- 1,023 | 51.5+/-9.2 | 0.035 +/- 0.005 | 2 |

As a control, activated myoblast populations of high purity grown in culture up to passage 3 provided re-colonization, as demonstrated by the nuclei expressing human lamin A/C counted on successive muscle sections throughout the muscle length, and as indicated by approximately 2-fold expansion ratio (Table 2). Approximately 60% of the human nuclei were found located beneath muscle fiber basal lamina, and several fibers contained human mitochondria expressing COX2, thus indicating that these cells directly participated to muscle formation, the other ones being identified within the endomysium. Several fibers expressing COX2 did not contain a human nucleus, at the level of a given cryostat section. Indeed, it is suggested that human mitochondria diffuse within the muscle fibers sarcolemma at longer distances than that simply defined by the plane of human nuclei. SMALD/34⁻ cells participated to muscle regeneration with similar or higher efficiency compared to myoblasts, since as few as 6 to 20 x 10³ cells generated 3 to 4 x 10⁴ human nuclei throughout the muscles (Figure 5 and Table 2). Therefore, each injected cell gave rise to approximately 3.5 nuclei, indicative of a proliferation step *in vivo.* This value may be largely underestimated, since cell death or disappearance upon injection generally decreases the efficacy of cell transplantation. Approximately half of the cells directly participated to muscle formation, as illustrated by their specific location, and the number of fibers expressing the mitochondrial COX2 (Figure 5). The remaining cells were identified within the endomysium. On the opposite, SMALD/34⁺ cells did not successfully integrate the recipient muscle tissue, as underlined by the small number of human cells identified by immunohistofluorescence (Figure 5) and consequently the poor ratio of labeled nuclei to initially injected cells (Table 2). Moreover, the surviving cells only contributed marginally to muscle formation, since only 3% of these were located beneath the basal lamina. Therefore, the phenotypical differences described above between SMALD/34⁻ and SMALD/34⁺ cells are traduced by functional differences *in vivo*.

### Longitudinal assessment of ALDH activity during cell culture

Freshly dissociated, FACS-phenotyped muscle cells were grown in myogenic proliferation medium and analyzed on successive passages for ALDH activity, expression of CD34, CD56 and CD15. As above, SMALD cells represented 2+/-1% of the total mononucleated cells upon dissociation. However, the inventors observed a dramatic increase in percentage of ALDH^{br} cells as soon as on the first passage, raising 78+/-11.5% of the cells. This percentage remained stable and tended to decrease until 54+/-30% on last passage, where it was significantly different from that on the first and second passages (Figure 6A). This increase cannot be simply explained by the exclusive proliferation of SMALD cells present on the day of seeding.
In this myogenic proliferation medium, CD34 phenotype was lost rapidly. Most cells rapidly expressed CD56, the marker of myoblasts, while an important proportion of CD56⁻ cells expressed CD15 which present mesenchymal-like characteristics. From the first passage, the percentage of ALDH^{br} cells in the CD56⁺ population (88+/-3%) was significantly higher than that in the CD56⁻ population (55+/-30%), and this difference persisted over passages (Figure 6B). These results suggest that ALDH expression is triggered in cell culture, to different extents according to the cell type.
In attempt to rely cell type and ALDH activity in culture, the sub-populations were sorted and grown in proliferation medium. The inventors observed that SNLALD/34⁻-derived cells, an almost pure CD56⁺ population, contained 86+/-4% of cells expressing high ALDH activity *in vitro.* On the opposite, SMALD/34⁺-derived cells, considered a pure CD56- population, contained 59+/-16% of cells expressing ALDH activity, the differences between these populations being significant (Figure 6C). These data confirm those observed in longitudinal culture studies of unsorted cells, with the CD56⁺ population harboring higher ALDH activity than the CD56⁻ one (Figure 6B).

### Discussion

### Identification of ALDH^{br} cells in human skeletal muscle

From classically dissociated muscle biopsies of various anatomical origins, the inventors directly (i.e., without any step of cell culture) identified, characterized and sorted two main populations of cells expressing high levels of ALDH (SMALD cells, which were SSC^{lo}), having features of stem or progenitor cells. The ALDH⁺ characteristic of these cells was abolished in the presence of excess of the specific inhibitor DEAB. SMALD cells represented approximately 3-4% of the mononucleated cells obtained upon enzymatic dissociation of the muscle biopsies. The use of fluorescent substrates of ALDH such as Aldefluor® offers several advantages: the methodology is not dependant upon the strategy for dissociation of the tissue and would not be sensitive to enzymatic surface detersion. It does not require cell permeabilization since Aldefluor® enters into the cells by diffusion. Upon oxidation, the fluorescent probe is trapped within the cells, thus allowing precise sorting of viable cells on the basis of expression levels, without altering their biological abilities. The selection relies robustly on a cellular function rather than on a membrane marker, whose turn-over may introduce biases. Analysis and selection do not require the use of intercalating agents, nor light excitation in the UV range nor any kind of selection medium. This methodology therefore allows the preparation of cell subsets of high purity, from as early as the dissociation step, which will prove useful to study the various proliferation and differentiation pathways followed by these cells. Finally, the reagent may be provided at clinical grade, and, as presented here, the methodology remains reliable using frozen and thawed stocks of dissociated cells, thus widening its usefulness in clinical perspectives.
CD34 labeling of dissociated muscle cells allowed further refining the characterization of SMALD populations. The inventors discriminated SMALD/34⁺ and SMALD/34' cells. The muscle biopsy also contained ALDH⁻/34⁺ (spCD34) and ALDH⁻/34⁻ (DN) cells. Being highly reproducible, the cell sorting could be standardized and yielded purities around 95%. These populations were clearly different, in terms of further expression of extracellular markers of commitment, and differentiation abilities in vitro and *in vivo.*

### SMALD cells belong to 2 sub-populations with distinct phenotypical and functional characteristics

While some markers were expressed by all populations at different levels, such as CD90, some were restricted to a given population, suggesting a specific stage of commitment (CD31, CD44, CD45, CD56, CD140b). Based on these distributions and the classical functional attributions of these markers, SMALD cells would be devoid of already engaged endothelial (CD31), hematopoietic (CD45) and satellite (CD56) cells. The lack of CD56⁺ labeling confirmed that SMALD cells were not primarily satellite cells, as observed on immunohistological sections. SMALD/34⁻ cells were enriched in CD44⁺ and CD105⁺ cells which are mesenchymal markers both involved in cell adhesion, migration and myogenesis comparatively to SMALD/34⁺ cells, which contained the marker of pericytes and mesenchymal stem cells CD140b. Noteworthy, these populations were still heterogeneous, or at different steps of maturation, since only a small percentage of cells was labeled for a given marker in each respective sub-population. CD133, which is expressed by important fractions of ALDH^{br} cells from other sources, was not detected on the (trypsin-treated) SMALD cells.
Upon culture, SMALD/34⁺ cells did not give rise to CD56⁺ cells, but mainly to an heterogenous population of CD56⁻ cells, they were unable to form myotubes but differentiated into adipogenic or osteogenic-like cells in respective differentiation media. Upon intramuscular injection, these cells survived poorly, remained within the endomysial compartment, and did not participate to muscle regeneration as witnessed by the lack of incorporation into muscle fibers.
Conversely, SMALD/34⁻ cells proliferated rapidly in the myogenic proliferation medium used in this study and rapidly expressed CD56, the marker of satellite cells and myoblasts. Upon differentiation, these cells fused and formed myotubes in myogenic medium, and osteoblastic-like cells in osteogenic medium, however they did not produce adipocytes in adipogenic medium. Finally, SMALD/34⁻ cells participated to muscle regeneration *in vivo* in a xenogenic context with high efficiency, as assessed by the presence of hundreds human nuclei within the SCID mouse muscle tissue, and the labeling of human COX2 protein inside muscle fibers. Indeed, when injected immediately after selection and without prior culture step, cells underwent proliferation, and more than half of them participated to muscle fiber formation. The efficacy of SMALD/34⁻ cells was equivalent or superior to that of standard human myoblasts produced in large-scale cell cultures.
Single positive CD34⁺ cells (spCD34) did not grow in culture in the myogenic proliferation medium, and were not able to participate efficiently to muscle regeneration *in vivo*. Double negative cells and unsorted cells provided variable amounts of CD56⁺ cells in culture. They participated very moderately to muscle regeneration when injected directly after selection.
The characterization of ALDH^{br} cells in the skeletal muscle tissue mandates their comparison with previously described ALDH^{br} cell lineages from other sources, or with known myogenic stem cells.

### Comparison between SMALD cells and ALDH^{br} cells from other sources

The comparison between ALDH^{br} expressing cells originating from hematopoietic compartments (bone marrow, peripheral blood, umbilical cord blood) and skeletal muscle reveals differences in absolute and relative numbers, the nature and proportions of associated markers, and the lineage differentiation abilities of the different fractions.
The frequency of SMALD cells is approximately ten to fifty times that of ALDH^{br} cells in peripheral blood. Therefore, SMALD cannot be simple passenger cells in the muscle tissue.
SMALD cells differ from BM and UCB cells, e.g., by the differential expression of CD45 and CD31. In the present study, CD45 was expressed by few DN cells, while it labels 65% and 99% of BM and PB ALDH^{br} cells, respectively. Here also, CD31 was expressed by spCD34 cells, not by SMALD cells, while it labels 99% of UCB cells and is depleted in BM ALDH^{br} fraction.
Most hematopoietic studies attributed the erythroid, lymphoid, myeloid or endothelial multilineage capacities to ALDH^{br}/CD34⁺, ALDH^{br}/CD133⁺ and/or ALDH^{br}/CD34⁺/CD133⁺ cells; some studies failed to detect significant hematopoietic activity in ALDH^{br}CD34⁻ fractions selected from UCB and PB. Meanwhile, no significant hematopoietic activity could be demonstrated in the ALDH^{br}/CD34⁻ population selected from UCB and PB. To the contrary, the results of the present inventors demonstrate that the myogenic capacities are the hallmark of SMALD/34⁻ cells, whereas SMALD/34+ cells do not have myogenic capacities.
The SMALD/34- cells further have osteogenic capacities, and the SMALD/34+ cells have osteogenic capacities, adipogenic capacities.
These observations suggest that ALDH^{br} populations differ in composition, function, and regenerative capacities depending on the tissue or fluid they are originating from.
The analysis of SMALD content of given muscle biopsies confers a predictive value as to the fate of long-term cultures in myogenic conditions. Indeed, increased ratio of SMALD/34⁻ to SMALD/34⁺ (corresponding to PP, predictor percentages) is indicative of the final yield of a given culture as for the final percentage of CD56⁺. This predictive percentage could be a very useful clinical tool, to predict the capacity of a biopsy to differentiate into CD56⁺ cells. This also suggests a role played by SMALD cells in myoblast production, in acute conditions of muscle degeneration such as that mimicked by dissociation and culture.

### Comparison between SMALD cells and classical myogenic progenitors

The comparison between SMALD and the various muscle-derived stem cells lineages also reveals fundamental differences in their biology, location, or nature of associated markers.

The results of the inventors demonstrate that SMALD are not classical satellite cells, since SMALD do not express CD56 and are not located beneath the muscle fibers basal lamina, and since satellite cells, are identified within the ALDH⁻/34⁻ muscle-derived fraction.
One may consider that SP and SMALD cells are both involved in detoxification. SP and SMALD cells are however involved in detoxification through different biological mechanisms. Moreover, muscle SP cells are heterogeneous and exclude drugs using ABCG2 transporters, while SMALD cells proceed to oxidation of biogenic or xenogenic aldehydes. Furthermore, the only overlap, if any, between the SP- and ALDH-defined cell populations in both BM- and UCB- derived cells was observed within the CD34⁺ cell population, which would then herein correspond to muscle ALDH^{br}/CD34⁺ cells. As herein demonstrated, the muscle ALDH⁻/CD34⁺ fraction does not undergo myogenic differentiation neither *in vitro* nor *in vivo.* Furthermore, the SP cells are not capable of myogenesis by themselves: they require co-culture with myoblasts to undergo myogenic specification. To the contrary, the SMALD cells undergo rapid myogenic differentiation (with CD56 expression) by themselves, in the absence of co-culture. Therefore, the SMALD cell population cannot correspond to the SP cell population, and defines a distinct cell population.

Human myo-endothelial cells initially harbor CD56⁺, CD34⁺, CD144⁺, CD31⁺ and CD45⁻, they account for 0.4% of the content of a dissociated muscle biopsy and participate efficiently to muscle regeneration *in vivo.* SMALD cells, either CD34⁺ or CD34', do not express CD56 nor CD31 and therefore, cannot be myo-endothelial cells. The fact that cells sharing myo-endothelial characteristics were observed in our single positive CD34 fraction (spCD34) upon sorting confirms this conclusion, although the ALDH⁻ CD34⁺ fraction did not proliferate in our specific myogenic culture conditions.
A muscle cell population expressing CD34⁺/CD133⁺ has been described as having myogenic capacities (Torrente *et al*., 2007). SMALD cells did not express CD133. Moreover, SMALD/34⁺ cells were unable to undergo myogenic differentiation. Therefore SMALD cannot be CD34⁺/CD133⁺ muscle progenitors. These CD34⁺/CD133⁺ cells may be contained in the present ALDH⁻ CD34⁺ fraction.
The direct comparison of the SMALD cells that are herein described to the human muscle mesangioblasts and pericytes in adult tissue is made difficult due to methodological differences. Indeed, while the present study focuses on freshly isolated cells, muscle mesangioblasts/pericytes are classically obtained through a cell culture step allowing cells to emigrate from within the muscle explants. Muscle pericytes have been described as uniformly negative for CD31, CD34, CD45 and CD133, weakly positive for CD90, CD105 and CD 146, and strongly positive for CD 13, CD44, ALP, CD 140b, and they were sorted on the basis of ALP⁺/CD56⁻ expressions. Freshly isolated SMALD are ALP-negative. Therefore, freshly isolated SMALD cells are distinct from and do not comprise any detectable proportion of pericytes. It does of course not preclude the fact that some particular sub-population of SMALD cells may express the phenotypes of pericytes upon *in vitro* growth.

### Conclusion and perspectives

In conclusion, this study for the first time demonstrates the presence of two populations of cells expressing high levels of ALDH within the human skeletal muscle tissue, thus providing a new tool for cell therapy developments, and mandating further investigations. The SMALD/34⁻ population presents the abilities to undergo myogenic differentiation *in vitro*, and to participate to muscle regeneration *in vivo*, thus adding a potential new player in the field of muscle homeostasis. However, the alternate differentiation capacities of the SMALD/34⁻ population along different lineages, and the physiological differentiation potentials of SMALD/34⁺ population, remain to be explored. Moreover, within the two large categories defined by CD34 expression, SMALD cell populations remain heterogeneous. Therefore the combined analysis of ALDH activity and complementary markers may allow defining several subsets having different roles in cellular ontogenic progression and tissue homeostasis. The present results prompt to further analyze the relationship between SMALD and ALDH^{br} cells prepared from other sources and the comparisons of their properties, with a special focusing on their migrating capacities.

### Example 2: Flow cytometric phenotyping of SMALD-derived cells in culture

In the present example, materials and methods are as described in example 1 above, unless otherwise specified.
Human SMALD/34- and SMALD/34+ cells were FACS-sorted, plated and grown in culture in myogenic medium and expanded up to the second or third passage as described in example 1 above.

Cells were detached using trypsin-EDTA, washed and labeled with various mAb conjugated to PE, APC, or FITC (BD Pharmingen, 1/20, 15 min at 4°C). The markers were: CD10, CD13, CD 15, CD31, CD34, CD44, CD45, CD49e, CD56, CD73, CD90, CD105, CD 106, CD 117, CD146, CD166. All mAb were IgG, except anti-CD15 (IgM). Non-specific fluorescence was determined using negative isotype controls (BD Pharmingen). In parallel, ALD expression was evaluated as described above.
Cells were analyzed by FACS (Facscalibur®, BD Pharmingen) using the Cell Quest® software. At least 10⁴ events were analyzed. Data are presented as mean +/- SE, n = 6.

The results are presented in Table 3 and in Figure 8.

**Table 3:**

| Markers | SMALD/34-(mean %) | SMALD/34+ (mean %) | SD SMALD/34- (%) | SD SMALD/34+ (%) |
|---|---|---|---|---|
| CD 56 | 92.81 | 0.12 | 5.63 | 0.22 |
| CD 15 | 0.35 | 47.97 | 0.46 | 20.2 |
| CD 73 | 96.81 | 91.89 | 3.18 | 9.06 |
| CD90 | 98.32 | 48.82 | 1.93 | 12.22 |
| CD 10 | 80.56 | 51.66 | 12.01 | 20.81 |
| CD 105 | 83.64 | 85.93 | 8.16 | 11.58 |
| CD 106 | 0.03 | 0.1 | 0.08 | 0.22 |
| CD 117 | 0 | 0 | 0 | 0 |
| CD13 | 96.82 | 96.99 | 3.1 | 4.05 |
| CD 146 | 76.88 | 10.2 | 15.99 | 15.23 |
| CD 166 | 85.35 | 80.04 | 10.13 | 19.05 |
| CD 31 | 0 | 0.13 | 0 | 0.08 |
| CD 34 | 0.08 | 0.04 | 0.13 | 0.1 |
| CD44 | 96.64 | 94.59 | 3.83 | 6.14 |
| CD 45 | 0.17 | 0.3 | 0.25 | 0.29 |
| CD 49e | 94.83 | 91.56 | 3.99 | 7.17 |
| ALD | 85.18 | 61.69 | 4.68 | 12.54 |

Significant differences between SMALD/34'- and SMALD/34⁺-derived cell populations were observed, notably regarding the percentage of labeled cells with the following markers: CD10, CD 15, CD56, CD90, CD 146, ALD activity.

### Example 3: Murine cells extraction and characterization

In the present example, materials and methods are as described in example 1 above, unless otherwise specified. Three young adult mice of C57Bl6XSVJ129 background were sacrificed by overdosage of anaesthetics (one 8-month and two 4-month old animals). The muscles of their lower limbs were harvested and pooled yielding 1.6 to 2.7 g of fresh tissue/mouse. Their hearts were harvested separately, yielding 0.2 to 0.3 g of fresh tissue.
All these murine tissues were minced and processed according to the same methodology as that used for preparing human muscle-derived cells. Each mouse was treated separately. Red cells were depleted from initial fresh bulk by incubation in lysis buffer (15 min, Beckman Coulter). Then cells were incubated in Aldefluor® assay buffer containing the ALDH substrate (StemCell, 1µM, 30 min, 37°C). Controls were obtained by prior incubation of cells with 50mM of the specific ALDH inhibitor diethylaminobenzaldehyde (DEAB). Then cells were centrifuged, suspended in Aldefluor® kit buffer, and analyzed under flow fluorocytometry (Facscalibur®, BD Pharmingen) using the Cell Quest® Software (10⁴ events analyzed), using the same settings as that used for the analysis of human cells (see example 1 above).
The results are presented in Figure 9 and in Table 4.

**Table 4:**

| | **% of ALDH+ cells** | |
|---|---|---|
| **Mice** | **Muscle** | **Heart** |
| A | 1.59 | 1.23 |
| B | 3.58 | 2.77 |
| C | 2.59 | 2.30 |
| Mean | 2.59 | 2.10 |
| SD | 1.00 | 0.79 |
| **Median** | **2.59** | **2.30** |

In both skeletal muscle-derived and heart muscle-derived samples, small populations of cells with low side-scatter (SSC^{lo}) and bright ALDH (ALDH^{br}) activity presented a shift of fluorescence in absence of DEAB, and disappeared in presence of the ALDH inhibitor DEAB. These characteristics were similar to that of human cells from skeletal muscle. The median percentage of this skeletal muscle ALDH^{br}/SSC^{lo} population (abbreviated SMALD) represented approximately 2-3% of the bulk mononucleated muscle cell suspension (2.59 +/- 1.00 % for skeletal muscle-derived cells, and 2.30 +/- 0.79 % for heart muscle-derived cells). These results indicate (1) that SMALD cells are present in murine (mouse) skeletal muscles, which expand their discovery to a second and distant mammal species, and (2) that SMALD cells are also present in murine (mouse) heart, suggesting a wide distribution of SMALD cells throughout the skeletal muscle compartments of the body. Given the tissue-specific myogenic differentiation ability of SMALD cells of skeletal muscle origin, a cardiomyogenic differentiation ability of the SMALD cells extracted from the heart muscle can be anticipated.

### BIBLIOGRAPHIC REFERENCES

Allessandri et al. 2004 "Isolation and culture of human muscle-derived stem cells able to differentiate into myogenic and neurogenic cell lineages." Lancet 364(9448): 1872-83.
Asakura et al. 2002, "Myogenic specification of side population cells in skeletal muscle", The Journal of Cell Biology, 159(1): 123-134.
Cai et al. 2004 "Membrane properties of rat embryonic multipotent neural stem cells", Journal of Neurochemistry 88: 212.
Corti et al. 2006a "Identification of a primitive brain-derived neural stem cell population based on aldehyde dehydrogenase activity" Stem Cells 24(4): 975-985.
Corti et al. 2006b "Transplanted ALDHhiSSClo neural stem cells generate motor neurons and delay disease progression of nmd mice, an animal model of SMARD1" Hum. Mol. Genet. 15(2): 167-187.
Ginestier et al. 2007 "ALDH1 is a Marker of Normal and Malignant Human Mammary Stem Cells and a Predictor of Poor Clinical Outcome ". Cell Stem Cell 1(5) :555-567.
Ham et al. 1988 "Improved media for normal human muscle satellite cells: serum-free clonal growth and enhanced growth with low serum" In vitro Cell Dev. Biol. 24: 833-844)
Hess et al. 2003 "Functional characterization of highly purified human hematopoietic repopulating cells isolated based on aldehyde dehydrogenase activity" Blood 102: 113a.
Jones, R. J., J. P. Barber, et al. 1995 "Assessment of aldehyde dehydrogenase in viable cells." Blood 85(10): 2742-6.
Kastan, M. B., E. Schlaffer, et al. 1990 "Direct demonstration of elevated aldehyde dehydrogenase in human hematopoietic progenitor cells." Blood 75(10): 1947-50.
Lombes A, Mendell JR, Nakase H, Barohn RJ, Bonilla E, Zevianni M, Yates AJ, Omerza J, Gales TL, Nakahara K, Rizzuto R, Engel WK, DiMauro S, 1989 "Myoclonic epilepsy and ragged-red fibers with cytochrome oxidase deficiency : neuropathology, biochemistry, and molecular genetics" Ann Neurol 26: 20-33.
Mitchell et al. 2006 "Immunophenotype of human adipose-derived cells: temporal changes in stromal-associated and stem cell-associated markers." Stem Cells 24(2): 376-85.
Nagano et al. 2007 "Identification of functional endothelial progenitor cells suitable for the treatment of ischemic tissue using human umbilical cord blood." Blood 110(1): 151-60.
Pittenger et al.1999 "Multilineage potential of adult human mesenchymal stem cells" Science 284: 143-147.
Storms, R. W., A. P. Trujillo, et al. 1999 "Isolation of primitive human hematopoietic progenitors on the basis of aldehyde dehydrogenase activity." Proc Natl Acad Sci U S A 96(16): 9118-23.
Torrente et al. 2007 "Autologous transplantation of muscle-derived CD133+ stem cells in Duchenne muscle patients." Cell Transplant 16(6): 563-77.
Vasiliou and Nebert 2005 Human Genomic 2:138-143.
WO 01/94555 and its US counter-part patent application(s) or patent(s) (e.g., US 2004 0043008 A1; US 2006 0088508 A1).

## Claims

1. A cell population obtainable by isolation from muscle tissue(s), wherein said cell population comprises at least 50%, preferably at least 60%, more preferably at least 70%, still more preferably at least 80%, still even more preferably at least 90%, most preferably at least 95%, still most preferably at least 98%, even still most preferably at least 99% of **ALDH**-positive cells,
and wherein the population of said ALDH-positive cells comprises:
- no more than 5%, preferably no more than 4.5%, more preferably no more than 4%, still more preferably no more than 3%, even still more preferably no more than 2%, most preferably no more than 1% of **CD45**-positive cells;
- no more than 5%, preferably no more than 4.5%, more preferably no more than 4%, still more preferably no more than 3%, even still more preferably no more than 2%, most preferably no more than 1% of **CD31**-positive cells;
- no more than 5%, preferably no more than 4.5%, more preferably no more than 4%, still more preferably no more than 3%, even still more preferably no more than 2%, most preferably no more than 1% of **ALP**-positive cells;
- no more than 5%, preferably no more than 4.5%, more preferably no more than 4%, still more preferably no more than 3%, even still more preferably no more than 2%, most preferably no more than 1% of **CD133-**positive cells.

2. The cell population of claim 1, wherein said ALDH is ALDH1 and/or ALDH3, more preferably ALDH1, most preferably ALDH1A1.

3. The cell population of any one of the preceding claims, wherein said population of ALDH-positive cells comprises no more than 5%, preferably no more than 4.5%, more preferably no more than 4%, still more preferably no more than 3%, even still more preferably no more than 2%, most preferably no more than 1% of **CD56-**positive cells.

4. The cell population of any one of the preceding claims, wherein said population of ALDH-positive cells comprises **CD90-**positive cell(s), preferably 0.4-50%, more preferably 0.45-48%, still more preferably 0.5-45%, most preferably 0.5-40% of CD90-positive cells.

5. The cell population of any one of the preceding claims, wherein said population of ALDH-positive cells comprises no more than 5%, preferably no more than 4.5%, more preferably no more than 4%, still more preferably no more than 3%, even still more preferably no more than 2%, most preferably no more than 1% of **CD146-**positive cells.

6. The cell population of any one of the preceding claims, wherein said population of ALDH-positive cells comprises no more than 5%, preferably no more than 4.5%, more preferably no more than 4%, still more preferably no more than 3%, most preferably no more than 2% of **CD140b-**positive cells.

7. The cell population of any one of the preceding claims, wherein said population of ALDH-positive cells comprises no more than 5%, preferably no more than 4.5%, more preferably no more than 4%, still more preferably no more than 3%, most preferably no more than 2% of **CD105-**positive cells.

8. The cell population of any one of the preceding claims, wherein said muscle tissue(s) comprises(comprise) striated muscle tissue, such as skeletal muscle tissue and/or cardiac muscle tissue, preferably endomysial tissue of skeletal muscle and/or cardiac muscle tissue, more preferably endomysial tissue of skeletal muscle.

9. The cell population of any one of the preceding claims, wherein said muscle tissue(s) comprises(comprise) human muscle tissue.

10. The cell population of any one of the preceding claims, wherein said ALDH-positive cells are human cells.

11. The cell population of any one of the preceding claims, which is obtainable by isolation from muscle tissue(s) without any step of *in vitro* culture, more particularly without any step of *in vitro* proliferation and/or differentiation culture.

12. The cell population of any one of the preceding claims, which is **capable of undergoing myogenic differentiation in the absence of co-cultured myoblasts or co-cultured cardiomyocytes.**

13. The cell population of any one of claims 1-12, wherein said ALDH-positive cells are **CD34-negative.**

14. The cell population of claim 13, wherein said population of ALDH-positive cells comprises no more than 2%, preferably no more than 1% of **CD140b-**positive cells.

15. The cell population of any one of claims 1-12, wherein said ALDH-positive cells are **CD34-positive.**

16. The cell population of claim 15, wherein said population of ALDH-positive cells comprises no more than 2%, preferably no more than 1% of **CD105-**positive cells.

17. A cell, which is obtainable by isolation from the cell population of any one of claims 1-16 without any step of *in vitro* culture, more particularly without any step of *in vitro* proliferation and/or differentiation culture, wherein said cell is ALDH-positive, CD45-negative, CD31-negative, ALP-negative, CD133-negative, CD56-negative, CD146-negative, CD105-negative, CD140b-negative.

18. The cell of claim 17, which is CD34-negative.

19. The cell of claim 17, which is CD34-positive.

20. The cell population of any one of claims 1-16 or the cell of any one of claims 17-19, for use as a cell population having differentiation capacities or as a cell having differentiation capacities, respectively.

21. The cell population of any one of claims 1-16 or the cell of any one of claims 17-19, for use as a cell population or cell having myogenic and/or adipogenic and/or osteogenic differentiation capacities.

22. The cell population of any one of claims 1-14, or the cell of claim 18, for use as a cell population or cell having myogenic and/or osteogenic differentiation capacities.

23. The cell population of any one of claims 1-12, 15-16, or the cell of claim 19, for use as a cell population or cell having adipogenic and/or osteogenic differentiation capacities.

24. A cell therapy product, comprising at least one cell population of any one of claims 1-16, or at least one cell of any one of claims 17-19, and optionally at least one pharmaceutically and/or physiologically acceptable vehicle.

25. The cell therapy product of claim 24, for use in the prevention and/or treatment and/or palliation of a muscular and/or neuromuscular disorder, and/or of an adipose tissue disorder, and/or of a bone and/or cartilage disorder.

26. A cell therapy product, comprising at least one cell population of any one of claims 1-14, or at least one cell of claim 18, and optionally at least one pharmaceutically and/or physiologically acceptable vehicle.

27. The cell therapy product of claim 26, for use in the prevention and/or treatment and/or palliation of a muscular and/or neuromuscular disorder, and/or of a bone and/or cartilage disorder.

28. A cell therapy product, comprising at least one cell population of any one of claims 1-12 and 15-16, or at least one cell of claim 19, and optionally at least one pharmaceutically and/or physiologically acceptable vehicle.

29. The cell therapy product of claim 28, for use in the prevention and/or treatment and/or palliation of an adipose tissue disorder, and/or of a bone and/or cartilage disorder.

30. The cell population of any one of claims 1-16, or the cell of any one of claims 17-19, for use as a cell population having differentiation capacities or as a cell having differentiation capacities, respectively, in the production of a differentiated cell therapy product or in the production of a tissue therapy product.

31. The cell population or cell of claim 30, wherein said therapy product is intended for the prevention and/or treatment and/or palliation of a muscular and/or neuromuscular disorder, and/or of an adipose tissue disorder, and/or of a bone and/or cartilage disorder.

32. The cell population of any one of claims 1-14, or the cell of claim 18, for use as a cell population having differentiation capacities or as a cell having differentiation capacities, respectively, in the production of a differentiated cell therapy product or in the production of a tissue therapy product, wherein said therapy product is intended for the prevention and/or treatment and/or palliation of a muscular and/or neuromuscular disorder, and/or of a bone and/or cartilage disorder.

33. The cell population of any one of claims 1-12, 15-16, or the cell of claim 19, for use as a cell population having differentiation capacities or as a cell having differentiation capacities, respectively, in the production of a differentiated cell therapy product or in the production of a tissue therapy product, wherein said therapy product is intended for the prevention and/or treatment and/or palliation of an adipose tissue disorder, and/or of a bone and/or cartilage disorder.

34. An *in vitro* process for the production of a cell population or of a cell having differentiation capacities, which comprises isolating a cell population as defined in any one of claims 1-16, or a cell as defined in any one of claims 17-19, from a sample of muscle tissue(s).

35. The process of claim 34, which does not comprise any step of *in vitro* culture of said isolated cell population or cell, more particularly any step of *in vitro* proliferation and/or differentiation culture of said isolated cell population or cell.

36. An *in vitro* process for the product of a muscular and/or neuromuscular tissue, and/or of an adipose tissue, and/or of a bone and/or cartilage, which comprises:
- the production of a cell population, or of a cell, in accordance with the process of claim 34,
- the *in vitro* culture of this cell population, or of this cell, under conditions favorable to and/or enabling the differentiation of cells from said cell population, or of said cell, into a muscular and/or neuromuscular tissue, and/or into an adipose tissue, and/or into a bone and/or cartilage.

37. *In vitro* use of the detection of ALDH to predict the differentiation potential of cells that are contained in a sample of muscle, preferably in a sample of skeletal muscle tissue and/or cardiac muscle tissue, preferably in a sample of skeletal muscle endomysium and/or cardiac muscle tissue, more preferably in a sample of skeletal muscle endomysium.

38. The *in vitro* use of claim 37, which further comprises the detection of CD34.
